(19) Europäisches Patentamt
European Patent Office
Office européen des brevets

(11) **EP 3 383 462 B1**

(12) **EUROPÄISCHE PATENTSCHRIFT**

(45) Veröffentlichungstag und Bekanntmachung des Hinweises auf die Patenterteilung:
**15.01.2020   Patentblatt 2020/03**

(21) Anmeldenummer: **16800886.0**

(22) Anmeldetag: **21.11.2016**

(51) Int Cl.:
*A61M 16/00* *(2006.01)*      *A61M 16/01* *(2006.01)*

(86) Internationale Anmeldenummer:
**PCT/EP2016/001953**

(87) Internationale Veröffentlichungsnummer:
**WO 2017/092851 (08.06.2017 Gazette 2017/23)**

(54) **BEATMUNGSVORRICHTUNG ZUR AUTOMATISIERTEN BEATMUNG EINES PATIENTEN**

VENTILATION DEVICE FOR PROVIDING AUTOMATED VENTILATION TO A PATIENT

DISPOSITIF DE VENTILATION POUR LA VENTILATION AUTOMATISÉE D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.12.2015   DE 102015015439**

(43) Veröffentlichungstag der Anmeldung:
**10.10.2018   Patentblatt 2018/41**

(73) Patentinhaber: **Drägerwerk AG & Co. KGaA**
**23558 Lübeck (DE)**

(72) Erfinder:
• **BUSCHKE, Wilfried**
**23560 Lübeck (DE)**
• **HÖRMANN, Christoph**
**3240 Mank (AT)**
• **MERSMANN, Stefan**
**23564 Lübeck (DE)**

(56) Entgegenhaltungen:
CN-A- 103 893 866      DE-A1-102011 106 406
US-A1- 2010 300 445    US-A1- 2011 041 850

**Beschreibung**

Aus dem Stand der Technik sind Beatmungsvorrichtungen sowie Verfahren bekannt, bei welchen eine druckgesteuerte oder eine druckunterstützende Beatmung eines Patienten vorgenommen wird.

Ferner ist es bekannt, im Rahmen solcher Beatmungsverfahren ein sogenanntes Weaning durchzuführen, bei welchem der Patient in einer sogenannten Komfortzone gehalten wird, wobei für den Zweck des Weaning im Rahmen einer druckunterstützenden Beatmung ein Solldruckwert bzw. ein Druckunterstützungswert in Abhängigkeit eines detektierten Tidalvolumens und eines endexspiratorischen Kohlendioxidkonzentrationswertes angepasst wird. Derartige Verfahren sind auch als sogenannte "Smart-Care/PS" Verfahren bekannt.

Aufgabe der vorliegenden Erfindung ist es, eine druckgesteuerte oder eine druckunterstützende Beatmung eines Patienten durchzuführen, wobei das Erreichen eines für den Patienten möglicherweise vorteilhaften Beatmungszustandes in einer verbesserten Art und Weise und automatisiert ermöglicht wird.

Die erfindungsgemäße Aufgabe wird gelöst durch eine Beatmungsvorrichtung nach dem Anspruch 1. Ferner wird die erfindungsgemäße Aufgabe gelöst durch eine Recheneinheit für eine Beatmungsvorrichtung zur automatisierten Beatmung eines Patienten nach dem Patentanspruch 12. Ferner wird die erfindungsgemäße Aufgabe gelöst durch ein Programm mit einem Programmcode zur Durchführung eines Verfahrens nach dem Patentanspruch 13.

Vorteilhafte Ausführungsformen der Erfindung sind Gegenstand der abhängigen Ansprüche und werden in der folgenden Beschreibung unter teilweiser Bezugnahme auf die Figuren näher erläutert.

Vorgeschlagen wird eine erfindungsgemäße Beatmungsvorrichtung zur automatisierten Beatmung eines Patienten, aufweisend einen exspiratorischen Port und einen inspiratorischen Port zum Anschluss eines dem Patienten zugewandten Beatmungsschlauches für ein Atemgas, eine Atemgasfördereinheit, wenigstens einen Volumenstromsensor zum Erfassen eines Volumenstroms des Atemgases, wenigstens einen Atemgassensor zum Erfassen einer Kohlendioxidkonzentration des Atemgases, wenigstens einen Drucksensor zum Erfassen eines Drucks des Atemgases, sowie wenigstens eine Recheneinheit, wobei die Recheneinheit dazu ausgebildet ist, die Atemgasfördereinheit in Abhängigkeit des erfassten Drucks und eines vorgegebenen Solldruckwertes anzusteuern, wobei die Recheneinheit ferner dazu ausgebildet ist, eine Anpassung des Solldruckwertes und eine Anpassung einer Beatmungsfrequenz in Abhängigkeit des erfassten Volumenstromes und in Abhängigkeit der erfassten Kohlendioxidkonzentration vorzunehmen.

Die erfindungsgemäße Beatmungsvorrichtung ist vorteilhaft, da die Recheneinheit dazu ausgebildet ist, nicht nur eine Anpassung des Solldruckwertes in Abhängigkeit eines erfassten Volumenstromes und in Abhängigkeit einer erfassten Kohlendioxidkonzentration vorzunehmen, sondern auch dazu ausgebildet ist, eine Anpassung der Beatmungsfrequenz in Abhängigkeit des erfassten Volumenstromes und in Abhängigkeit der erfassten Kohlendioxidkonzentration vorzunehmen. Hierdurch wird möglicherweise eine verbesserte Form der Beatmung des Patienten im Rahmen der automatisierten Beatmung erreicht, welche vorzugsweise eine druckgesteuerte oder eine druckunterstützende Beatmung ist. Die Beatmung wird also nicht nur hinsichtlich des Solldruckwertes sondern auch hinsichtlich einer zu berücksichtigenden Beatmungsfrequenz verändert.

Vorzugsweise ist die Recheneinheit dazu ausgebildet, die Atemgasfördereinheit in Abhängigkeit des erfassten Drucks, des vorgegebenen Solldruckwertes und ferner der Beatmungsfrequenz anzusteuern, wobei die Recheneinheit ferner dazu ausgebildet ist, die Atemgasfördereinheit so anzusteuern, dass die automatisierte Beatmung als eine druckgesteuerte Beatmung durchgeführt wird.

Diese Ausgestaltung der Erfindung ist vorteilhaft, da im Rahmen einer druckgesteuerten Beatmung die Beatmungsfrequenz für die Ansteuerung der Atemgasfördereinheit durch die Recheneinheit herangezogen wird.

Vorzugsweise ist die Recheneinheit dazu ausgebildet, die Atemgasfördereinheit so anzusteuern, dass die automatisierte Beatmung als eine druckunterstützende Beatmung durchgeführt wird, wobei die Recheneinheit dazu ausgebildet ist, in Abhängigkeit der Beatmungsfrequenz eine Ausgabe eines Warnsignals zu steuern. Diese Ausgestaltung der Erfindung ist vorteilhaft, da die Ausgabe des Warnsignals in Abhängigkeit der Beatmungsfrequenz davon abhängt, in welcher Weise die Recheneinheit zuvor die Beatmungsfrequenz in Abhängigkeit des erfassten Volumenstromes und in Abhängigkeit der erfassten Kohlendioxidkonzentration angepasst hat. Ein solches Warnsignal kann dann durch einen Kliniker möglicherweise als Hinweis dienen, damit der Kliniker ggf. eine Änderung der automatisierten Beatmung in Betracht zieht.

Vorzugsweise ist die Recheneinheit dazu ausgebildet, auf Basis des erfassten Volumenstroms ein dem Patienten zugeführtes Tidalvolumen zu bestimmen, ferner auf Basis der erfassten Kohlendioxidkonzentration eine endexspiratorische Kohlendioxidkonzentration et $CO_2$ zu bestimmen und die Anpassung des Solldruckwertes sowie die Anpassung der Beatmungsfrequenz in Abhängigkeit des bestimmten Tidalvolumens und der bestimmten endexspiratorischen Kohlendioxidkonzentration vorzunehmen. Vorzugsweise ist hierbei die Recheneinheit dazu ausgebildet ist, die Anpassung des Solldruckwertes und die Anpassung der Beatmungsfrequenz in Abhängigkeit des bestimmten Tidalvolumens, eines Zieltidalvolumens, der bestimmten endexspiratorischen Kohlendioxidkonzentration und einer Ziel-Kohlendioxidkonzentration vorzunehmen. Hierdurch wird eine Regelung des Tidalvolumens und der endexspiratorischen Kohlendioxidkonzentration auf die jeweiligen Zielwerte erreicht.

Vorzugsweise ist die Recheneinheit dazu ausgebildet, die Anpassung des Solldruckwertes und die Anpassung der Beatmungsfrequenz in Abhängigkeit des bestimmten Tidalvolumens, eines oberen Volumengrenzwertes, eines unteren Volumengrenzwertes, der bestimmten endexspiratorischen Kohlendioxidkonzentration, eines oberen Konzentrationsgrenzwertes und eines unteren Konzentrationsgrenzwertes vorzunehmen. Diese Ausgestaltungen der Erfindung ist vorteilhaft, da durch die jeweiligen Grenzwerte bezogen auf Tidalvolumen und endexspiratorische Kohlendioxidkonzentration eine sogenannte Komfortzone zur Beatmung des Patienten definiert wird, wobei durch Vergleich dieser Komfortzone und dem Tidalvolumen einerseits als auch der der endexspiratorischen Kohlendioxidkonzentration die Anpassung der Beatmungsfrequenz und des Solldruckwertes vorgenommen wird, um den Patienten während der Beatmung innerhalb dieser Komfortzone zu behalten. Diese Komfortzone kann ggf. für den Patienten vorteilhaft sein bzw. eine möglicherweise adäquate Beatmung darstellen. Dieses wird dadurch erreicht, dass eine Anpassung der Beatmungsfrequenz und des Solldruckwertes in Abhängigkeit von zwei Parametern, nämlich Tidalvolumen und endexspiratorischer Kohlendioxidkonzentration, sowie der definierten Komfortzone, gegeben durch die Grenzwerte, durchgeführt wird.

Vorzugsweise ist die Recheneinheit dazu ausgebildet, die Atemgasfördereinheit in Abhängigkeit des erfassten Drucks, des vorgegebenen Solldruckwertes und ferner der Beatmungsfrequenz anzusteuern, ferner die Atemgasfördereinheit so anzusteuern, dass die automatisierte Beatmung als eine druckgesteuerte Beatmung durchgeführt wird, ferner eine Spontanatemaktivität des Patienten auf Basis des erfassten Volumenstroms zu detektieren und schließlich die Konzentrationsgrenzwerte in Abhängigkeit des Detektionsergebnisses anzupassen. Diese Ausgestaltung der Erfindung ist vorteilhaft, da im Zuge einer druckgesteuerten Beatmung möglichen und ggf. unerwünschten Spontanatemversuchen des Patienten durch eine Anpassung der Konzentrationsgrenzwerte möglicherweise entgegengewirkt werden kann.

Vorzugsweise ist die Recheneinheit dazu ausgebildet, den oberen Volumengrenzwert, den unteren Volumengrenzwert, den oberen Konzentrationsgrenzwert und den unteren Konzentrationsgrenzwert in Abhängigkeit einer Vorgabe hinsichtlich einer Lungeneigenschaft des Patienten zu wählen. Diese Ausgestaltung ist vorteilhaft, da die mittels der Grenzwerte definierte Komfortzone eine Lungeneigenschaft eines Patienten berücksichtigen kann. Diese Lungeneigenschaft kann durch eine Vorgabe eines Klinikers vorgegeben bzw. eingegeben werden.

Vorzugsweise ist die Recheneinheit dazu ausgebildet, den oberen Konzentrationsgrenzwert und den unteren Konzentrationsgrenzwert ferner in Abhängigkeit einer Vorgabe hinsichtlich einer erwünschten Gasaustauschrate des Patienten zu wählen. Diese Ausgestaltung ist vorteilhaft, da eine Definition der Komfortzone mittels der Konzentrationsgrenzwerte in Abhängigkeit einer Vorgabe eines Klinikers hinsichtlich einer erwünschten Gasaustauschrate eines Patienten gewählt werden kann. Eine derartige Gasaustauschrate ist beispielsweise durch eine sogenannte normale Ventilation eines Patienten oder aber beispielsweise eine milde Hyperventilation eines Patienten gekennzeichnet. Daher ist eine Gasaustauschrate mit anderen Worten ein Ventilationsgrad bzw. ein Beatmungsgrad eines Patienten.

Vorzugsweise ist der obere Volumengrenzwert ein erster oberer Volumengrenzwert, wobei ferner der untere Volumengrenzwert ein erster unterer Volumengrenzwert ist, wobei die Recheneinheit ferner dazu ausgebildet ist, die Anpassung des Solldruckwertes und die Anpassung der Beatmungsfrequenz in Abhängigkeit eines zweiten oberen Volumengrenzwertes und eines zweiten unteren Volumengrenzwertes vorzunehmen. Diese Ausgestaltung der Erfindung ist vorteilhaft, da die Anpassung des Solldruckwertes sowie die Anpassung der Beatmungsfrequenz nicht nur starr anhand jeweiliger erster oberer und erster unterer Grenzwerte bezogen auf das Tidalvolumen vorgenommen wird, sondern dass auch ein Grad einer Abweichung des Tidalvolumens von den ersten Grenzwerten anhand der Berücksichtigung der zweiten Volumengrenzwerte für die Anpassung berücksichtigt werden kann.

Vorzugsweise ist der obere Konzentrationsgrenzwert ein erster oberer Konzentrationsgrenzwert, wobei ferner der untere Konzentrationsgrenzwert ein erster unterer Konzentrationsgrenzwert ist, wobei die Recheneinheit ferner dazu ausgebildet ist, die Anpassung des Solldruckwertes und die Anpassung der Beatmungsfrequenz in Abhängigkeit eines zweiten oberen Konzentrationsgrenzwertes und eines zweiten unteren Konzentrationsgrenzwertes vorzunehmen. Diese Ausgestaltung der Erfindung ist vorteilhaft, da die Anpassung des Solldruckwertes sowie die Anpassung der Beatmungsfrequenz nicht nur starr anhand jeweiliger erster oberer und erster unterer Grenzwerte bezogen auf die endexspiratorische Kohlendioxidkonzentration vorgenommen wird, sondern dass auch ein Grad einer Abweichung der endexspiratorischen Kohlendioxidkonzentration von den ersten Grenzwerten anhand der zweiten Konzentrationsgrenzwerte für die Anpassung berücksichtigt werden kann.

Ein entsprechendes nicht erfindungsgemäßes Verfahren zur automatisierten Beatmung eines Patienten, weist die Schritte auf: Zuführen eines Atemgases zu einem Patienten über einen inspiratorischen Port und Rückführen des Atemgases über einen exspiratorischen Port durch Betreiben einer Atemgasfördereinheit, Erfassen eines Volumenstroms des Atemgases mittels wenigstens eines Volumenstromsensors, Erfassen einer Kohlendioxidkonzentration des Atemgases mittels wenigstens eines Atemgassensors, Erfassen eines Drucks des Atemgases mittels wenigstens eines Drucksensors, Ansteuern der Atemgasfördereinheit in Abhängigkeit des erfassten Drucks und eines vorgegebenen Solldruckwertes mittels wenigstens einer Recheneinheit, sowie eine Anpassung des Solldruckwertes und eine Anpassung einer Beatmungsfrequenz in Abhängigkeit des erfassten Volumenstromes und in Abhängigkeit der erfassten Kohlendioxidkonzentration mittels der Recheneinheit.

Vorgeschlagen wird ferner eine Recheneinheit für eine Beatmungsvorrichtung zur automatisierten Beatmung eines Patienten, welche ausgebildet ist zum: Erfassen eines Volumenstromsignals, welches einen Volumenstrom eines Atemgases indiziert, Erfassen eines Kohlendioxidkonzentrationssignals, welches eine Kohlendioxidkonzentration des Atemgases indiziert, Erfassen eines Drucksignals, welches einen Druck des Atemgases indiziert sowie Bereitstellen eines Ansteuersignals für eine Atemgasfördereinheit . Die Recheneinheit ist ausgebildet, das Ansteuersignal in Abhängigkeit des erfassten Drucksignals und eines vorgegebenen Solldruckwertes zu bestimmen sowie eine Anpassung des Solldruckwertes und eine Anpassung einer Beatmungsfrequenz in Abhängigkeit des erfassten Volumenstromsignals und in Abhängigkeit des erfassten Kohlendioxidkonzentrationssignals vorzunehmen.

Ein entsprechendes nicht erfindungsgemäßes Verfahren zum Betreiben einer Beatmungsvorrichtung zur automatisierten Beatmung eines Patienten, weist die Schritte auf: Erfassen eines Volumenstromsignals, welches einen Volumenstrom eines Atemgases indiziert, Erfassen einer Kohlendioxidkonzentrationssignals, welches eine Kohlendioxidkonzentration des Atemgases indiziert, Erfassen eines Drucksignals, welches einen Druck des Atemgases indiziert, Bereitstellen eines Ansteuersignals für eine Atemgasfördereinheit in Abhängigkeit des erfassten Drucksignals und eines vorgegebenen Solldruckwertes sowie eine Anpassung des Solldruckwertes und eine Anpassung einer Beatmungsfrequenz in Abhängigkeit des erfassten Volumenstromsignals und in Abhängigkeit des erfassten Kohlendioxidkonzentrationssignals. Vorzugsweise wird dieses Verfahren mit Computerprogrammmitteln auf wenigstens einer Recheneinheit ausgeführt wird. Vorgeschlagen wird ferner ein Programm mit einem Programmcode zur Durchführung des zuvor genannten Verfahrens, wenn der Programmcode auf einem Computer, einem Prozessor oder einer programmierbaren Hardwarekomponente ausgeführt wird.

Vorteile der vorgeschlagenen Beatmungsvorrichtung gelten ebenso für das entsprechende nicht erfindungsgemäße Verfahren zur automatisierten Beatmung eines Patienten. Ebenso gelten diese Vorteile für die vorgeschlagene Recheneinheit für eine Beatmungsvorrichtung zur automatisierten Beatmung eines Patienten. Ebenso gelten diese Vorteile auch für das entsprechende nicht erfindungsgemäße Verfahren zum Betreiben einer Beatmungsvorrichtung zur automatisierten Beatmung und auch für das vorgeschlagene Programm mit einem Programmcode.

Im Folgenden wird die Erfindung anhand spezieller Ausführungsformen ohne Beschränkung des allgemeinen Erfindungsgedankens anhand der Figuren näher erläutert. Dabei zeigen:

Fig. 1      einen zeitlichen Druckverlauf sowie einen zeitlichen Volumenstromverlauf im Zuge einer Inspiration und einer Exspiration,

Fig. 2      einen zeitlichen Druckverlauf sowie einen zeitlichen Volumenstromverlauf im Rahmen einer druckgesteuerten Beatmung, bei welcher keine Spontanatemversuche eines Patienten ermöglicht werden sollen,

Fig. 3      einen zeitlichen Druckverlauf sowie ein zeitlichen Volumenstromverlauf im Rahmen einer druckunterstützenden Beatmung, welche im Rahmen von Spontanatemversuchen eines Patienten erfolgt,

Fig. 4      einen zeitlichen Druckverlauf sowie ein zeitlichen Volumenstromverlauf im Rahmen einer druckgesteuerten Beatmung, bei welcher Spontanatemversuche eines Patienten zugelassen werden,

Fig. 5      eine erfindungsgemäße Beatmungsvorrichtung,

Fig. 6      Verfahrensschritte zur Initialisierung und Initiierung verschiedener Ausführungsformen eines nicht erfindungsgemäßen Verfahrens,

Fig. 7      Verfahrensschritte gemäß einer ersten Ausführungsform eines nicht erfindungsgemäßen Verfahrens,

Fig. 8      eine Illustration von Grenzwerten,

Fig. 9      Verfahrensschritte gemäß einer zweiten Ausführungsform eines nicht erfindungsgemäßen Verfahrens,

Fig. 10      Verfahrensschritte gemäß einer dritten Ausführungsform eines nicht erfindungsgemäßen Verfahrens,

Fig. 11      eine Tabelle mit durch einen Kliniker vorgebbaren Vorgabewerten,

Fig. 12a, b      jeweilige Tabellen mit Grenzwerten zur Durchführung des nicht erfindungsgemäßen Verfahrens gemäß der ersten bzw. der zweiten Ausführungsform,

Fig. 13    eine Tabelle mit Grenzwerten zur Durchführung des nicht erfindungsgemäßen Verfahrens gemäß der dritten Ausführungsform,

Fig. 14    jeweilige Tabellen zur Bestimmung von Ventilationsgraden bzw. Gasaustauschraten bezogen auf ein Tidalvolumen bzw. eine endexspiratorische Kohlendioxidkonzentration,

Fig. 15    eine Tabelle mit Werten zur Anpassung des Solldruckwertes und zur Anpassung der Beatmungsfrequenz,

Fig. 16    Teilverfahrensschritte zur Anpassung von Konzentrationsgrenzwerten,

Fig. 17    bevorzugte alternative Teilverfahrensschritte für den Fall, dass auf ein Zieltidalvolumen und auf eine Ziel-Kohlendioxidkonzentration abgestellt wird.

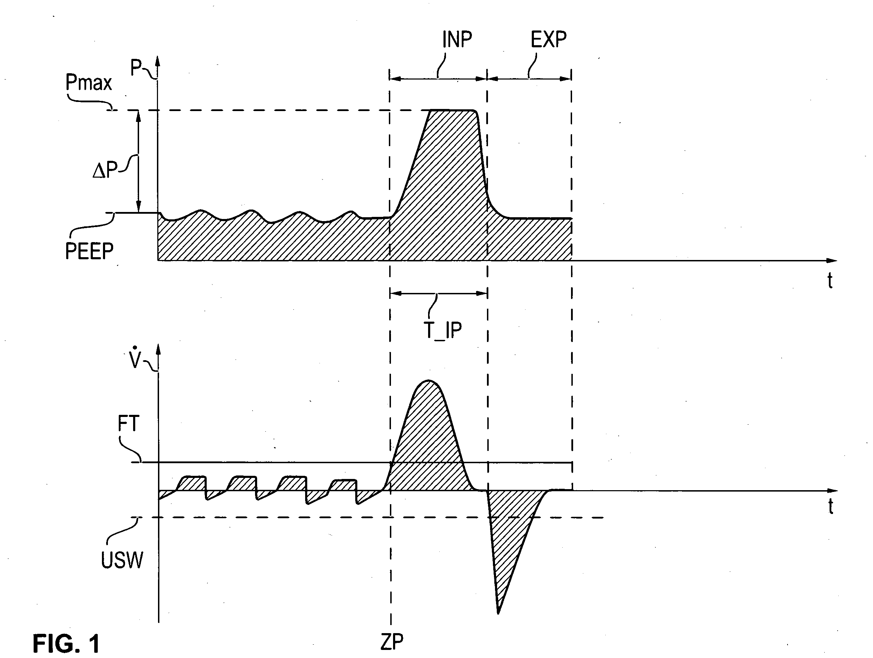

Figuren 1 bis 4 zeigen aus dem Stand der Technik bekannte Kurvenverläufe, anhand derer nun die Prinzipien einer Triggersteuerung, einer druckunterstützenden Beatmung, einer druckgesteuerten Beatmung ohne Zulassen von Spontanatemversuchen sowie einer druckgesteuerten Beatmung mit Zulassen von Spontanatemversuchen erläutert werden. Diese Prinzipien sind auch dem Dokument

-    "Beatmungsmodi in der Intensivmedizin", Karin Deden, Dräger Medical GmbH

entnehmbar.

Prinzipien der druckgesteuerten Beatmung sowie der druckunterstützenden Beatmung sind auch aus dem Dokument

-    Gebrauchsanweisung "Zeus Infinity Empowered", Dräger Medical AG & Co. KG, 1. Ausgabe, Februar 2009

entnehmbar.

Die Figur 1 zeigt zur Illustration einer Triggersteuerung einen Druckverlauf eines Druckwertes P über der Zeit, wobei ferner ein zeitlicher Verlauf eines Volumenstroms V zu sehen ist. Wird ein Patient durch eine Beatmungsvorrichtung beatmet, so erfolgt die Drucksteuerung derart, dass vor einer inspiratorischen Phase INP der Druck auf einen mindesten endexspiratorischen Druck PEEP (Positive and Exspiratory Pressure) geregelt wird. Macht ein Patient einen Spontanatemversuch, so führt dies zum Überschreiten des Volumenstroms V über eine sogenannte Triggerschwelle bzw. Flow-Triggerschwelle FT zum Zeitpunkt ZP. Es wird dann bei Schwellenüberschreitung der Druck P derart geregelt, dass der Druck P auf einen Maximaldruck Pmax geregelt wird, wobei dieser Maximaldruck Pmax um einen Differenzdruck $\Delta P$ über dem Mindestdruck PEEP liegt. Üblicherweise wird eine Zeitdauer T_IP für eine inspiratorische Phase vorgegeben, sodass dann nach Ablauf der Zeitdauer T_IP mit der exspiratorischen Phase EXP begonnen wird, während welcher wiederum der Druck P auf den Minimaldruck PEEP abgesenkt wird. Somit ergibt sich während der exspiratorischen Phase aufgrund des Ausströmens des Volumenstromes V aus dem Patienten heraus ein negativer Volumenstrom V.

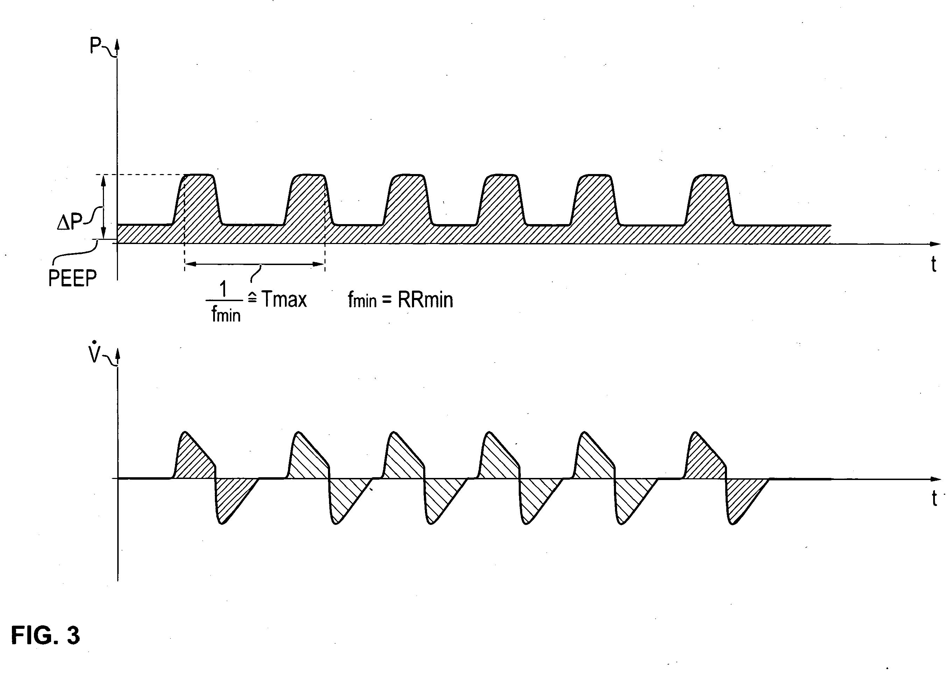

Eine derartige, triggergesteuerte Beatmung wird üblicherweise im Rahmen von einer druckunterstützenden Beatmung durchgeführt, wie sie auch noch einmal in der Figur 3 gezeigt ist. Eine solche druckunterstützende Beatmung wird ferner vorzugsweise derart durchgeführt, dass der Patient aufgrund seiner durch ihn initiierten Triggerung eine Mindestatemfrequenz fmin bzw. RRmin herbeiführen muss, sodass sich ein maximales Zeitfenster Tmax zwischen zwei Anfangszeitpunkten benachbarter inspiratorischer Phase ergibt. Führt die durch den Patienten getriggerte Beatmung dazu, dass nach Ablauf des Zeitfensters Tmax keine erneute Inspiration durchgeführt wird, bzw. ist seine eigene Atemfrequenz geringer als die Mindestatemfrequenz RRmin, so kann in einem solchen Fall eine Warnung ausgegeben werden. Eine solche Warnung kann ein Kliniker als Hinweis werten, ggf. eine Änderung der automatisierten Beatmung in Betracht zu ziehen.

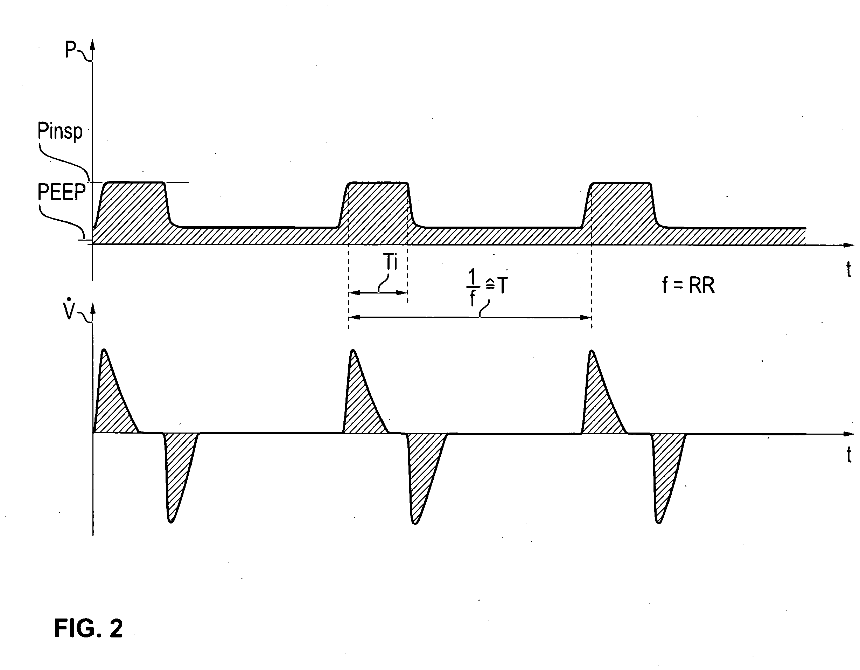

Die Figur 2 zeigt einen zeitlichen Druckverlauf P sowie einen zeitlichen Volumenstromverlauf V für den Fall einer druckgesteuerten Beatmung, bei welcher keine Spontanatemversuche eines Patienten zugelassen werden. Hierbei wird eine feste Zeitdauer Ti für eine inspiratorische Phase vorgegeben sowie eine Beatmungsfrequenz f bzw. RR. Die Frequenz f bzw. RR definiert hierbei den zeitlichen Abstand T zwischen zwei Anfängen jeweiliger benachbarter inspiratorischer Phasen. Beginnt eine inspiratorische Phase, so wird der Druck ausgehend vom dem Mindestwert PEEP auf einen Maximaldruckwert bzw. Solldruckwert Pinsp gesteuert bzw. geregelt.

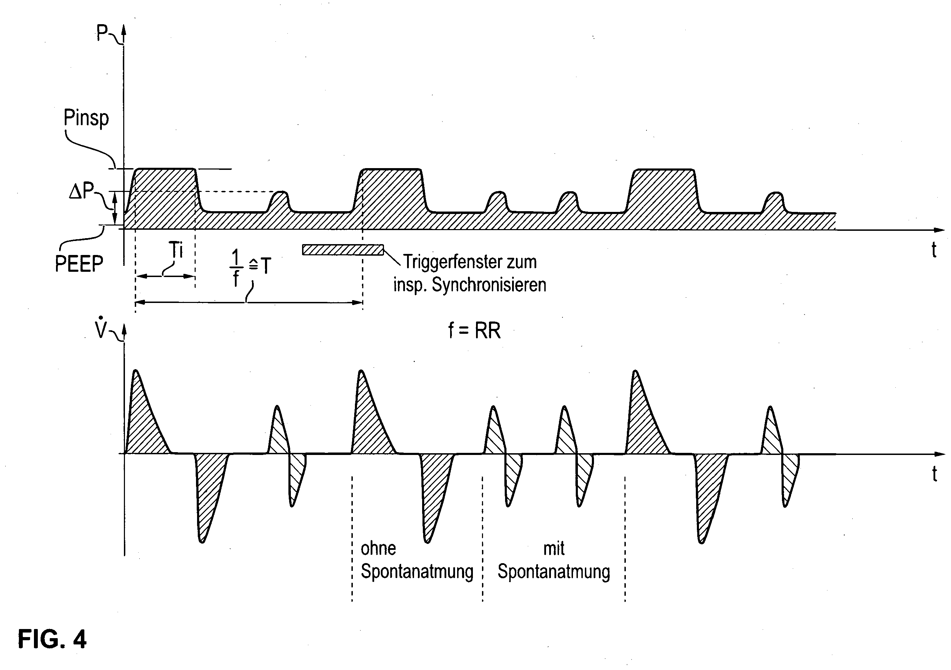

Die Figur 4 zeigt das Prinzip einer druckgesteuerten Beatmung, bei welcher auch Spontanatemversuche eines Patienten mit Flowtriggerung durch den Patienten für eine Druckunterstützung zugelassen werden. Es ergeben sich also inspiratorische Phasen mit zuvor erläuterter Drucksteuerung, in denen der Druck P auf den Solldruckwert Pinsp gesteuert wird. Ferner gibt es inspiratorische Phasen mit Druckunterstützung aufgrund durch den Patienten verursachter Triggerung aufgrund von Spontanatmung, bei welchen eine Unterstützung bzw. Steuerung des Druckwertes auf ein Druckniveau erfolgt, welches um die Druckdifferenz ∆P oberhalb des Mindestdrucks PEEP liegt.

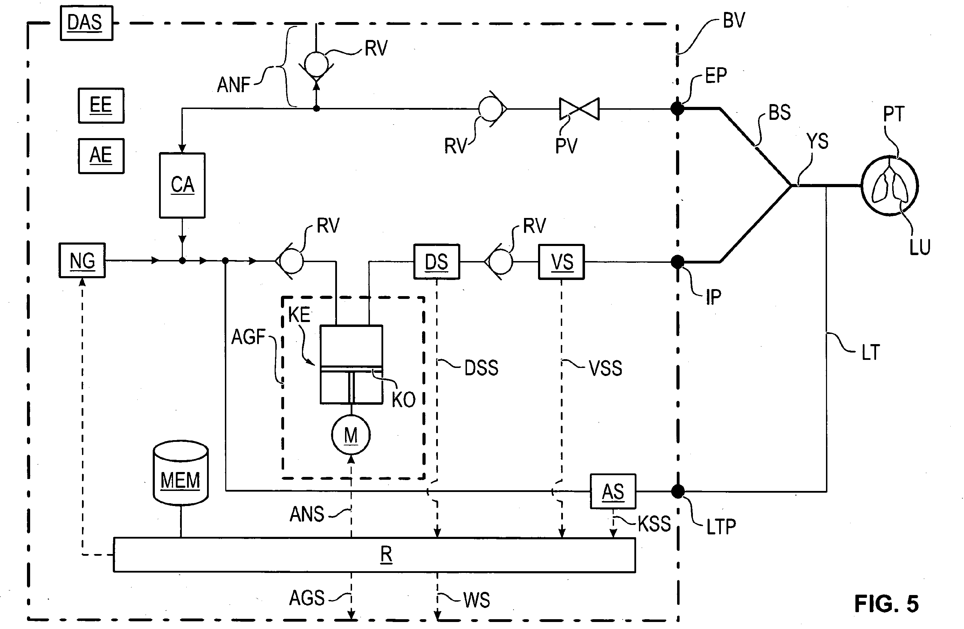

Die Figur 5 zeigt die erfindungsgemäße Vorrichtung BV zur automatisierten Beatmung eines Patienten PT. Die erfindungsgemäße Beatmungsvorrichtung BV weist einen inspiratorischen Port IP und einen exspiratorischen Port EP auf, an welche ein Beatmungsschlauch BS angeschlossen werden kann, welcher dem Patienten PT zugewandt ist. Über diesen Beatmungsschlauch BS wird ein Atemgas dem Patienten zugeführt und auch wieder von dem Patienten hin zur Vorrichtung BV abgeführt. Die Zuführung erfolgt über den inspiratorischen Port IP, die Abführung erfolgt über den exspiratorischen Port EP. Der Beatmungsschlauch BS führt die Anschlüsse der Ports EP, IP an einem sogenannten Y-Stück YS zusammen, welches dann üblicherweise an einem Tubus endet, der dem Patienten PT eingeführt wird, um ihn über seine Lunge LU zu beatmen.

Die Beatmungsvorrichtung BV weist ferner eine Atemgasfördereinheit AGF auf. Vorzugsweise ist die Atemgasfördereinheit AGF eine Kolbeneinheit KE, in welcher ein Kolben KO durch einen Motor M vor- bzw. zurückgeführt werden kann.

Die Beatmungsvorrichtung BV weist wenigstens einen Volumenstromsensor VS zum Erfassen eines Volumenstroms des Atemgases auf. Der Volumenstromsensor VS kann ein Volumenstromsensorsignal VSS an eine Recheneinheit R bereitstellen. Bei der Recheneinheit R handelt es sich um wenigstens eine Recheneinheit, welche auch durch einen Verbund mehrerer Recheneinheiten verwirklicht werden kann.

Ferner weist die Beatmungsvorrichtung BV einen Drucksensor DS zum Erfassen eines Drucks des Atemgases auf. Der Drucksensor DS stellt ein Drucksensorsignal DSS an die Recheneinheit R bereit.

Ein Mindestdruck PEEP wird vorzugsweise durch ein Ventil PV, welches vorzugsweise in dem Bereich des exspiratorischen Ports EP liegt, realisiert.

Die Beatmungsvorrichtung BV weist ferner einen Atemgassensor AS zum Erfassen einer Kohlendioxidkonzentration des Atemgases auf. Der Sensor AS ist vorzugsweise hinter einer Messleitung LT vorgesehen, welche eine Messprobe des Atemgases am Y-Stück YS abführt und an einen Messgasport LTP angeschlossen ist. Der Atemgassensor stellt ein Kohlendioxidkonzentrationssignal KSS an die Recheneinheit R bereit. Die Recheneinheit R ist dazu ausgebildet, über ein Ansteuersignal ANS die Atemgasfördereinheit AGF anzusteuern.

Für den bevorzugten Fall, dass die Beatmungsvorrichtung BV eine Anästhesiebeatmungsvorrichtung ist, weist die Beatmungsvorrichtung BV vorzugsweise einen Kohlendioxidabsorber CA sowie eine Narkosegasmischeinheit NG auf. Über die Narkosegasmischeinheit NG kann dann ein für die Narkose erforderliches Gasgemisch in den Atemkreislauf eingebracht werden. Ferner weist die Beatmungsvorrichtung BV als Anästhesiebeatmungsvorrichtung vorzugsweise eine Anästhesiegasfortleitung ANF bzw. einen Anschluss an eine Anästhesiegasfortleitung ANF auf. Der Gasfluss innerhalb der Beatmungsvorrichtung BV ist vorzugsweise durch Rückschlagventile RV kontrolliert. Die Recheneinheit R steuert vorzugsweise die Narkosegasmischeinheit NG mittels eines Steuersignals NGAS.

Die Beatmungsvorrichtung BV aus Figur 5 weist vorzugsweise eine Eingabeeinheit EE bzw. eine Schnittstelle zu einer Eingabeeinheit EE auf, mittels welcher Eingaben an der Beatmungsvorrichtung BV entgegengenommen werden können, welche durch einen Bediener bzw. Kliniker vorgenommen werden können.

Die Recheneinheit R greift vorzugsweise auf eine Speichereinheit MEM zurück, um die nicht erfindungsgemäßen Verfahren durchzuführen.

Die Recheneinheit R kann ferner vorzugsweise ein Warnsignal WS ausgeben. Dieses erfolgt vorzugsweise über eine Datenschnittstelle DAS der Vorrichtung BV.

Die erfindungsgemäße Beatmungsvorrichtung ist dazu ausgebildet, eine druckgesteuerte oder eine druckunterstützende Beatmung des Patienten PT durchzuführen.

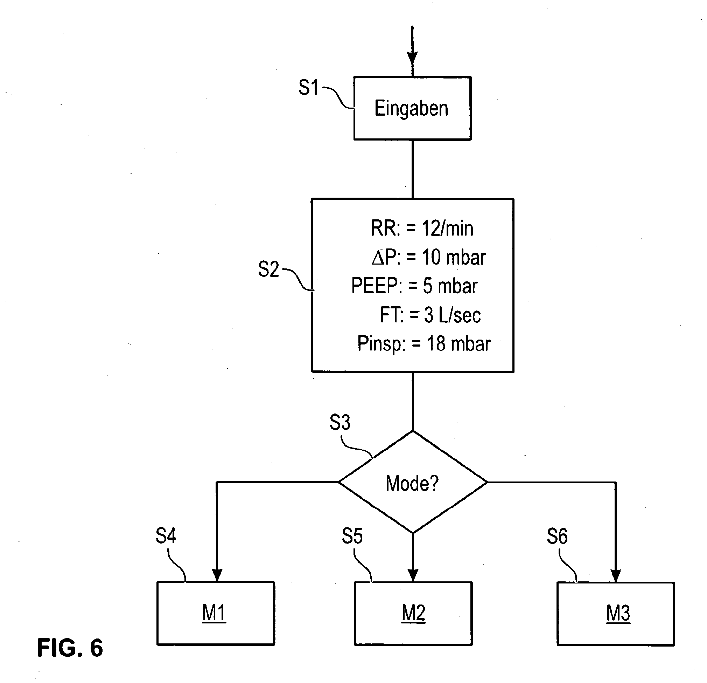

Figur 6 zeigt Initiationsschritte, mittels derer die Beatmungsvorrichtung aus Figur 5 zur Durchführung verschiedener Ausführungsformen eines nicht erfindungsgemäßen Verfahrens veranlasst werden kann. In einem Schritt S1 erfolgt eine Eingabe von Vorgaben durch einen Nutzer bzw. Kliniker.

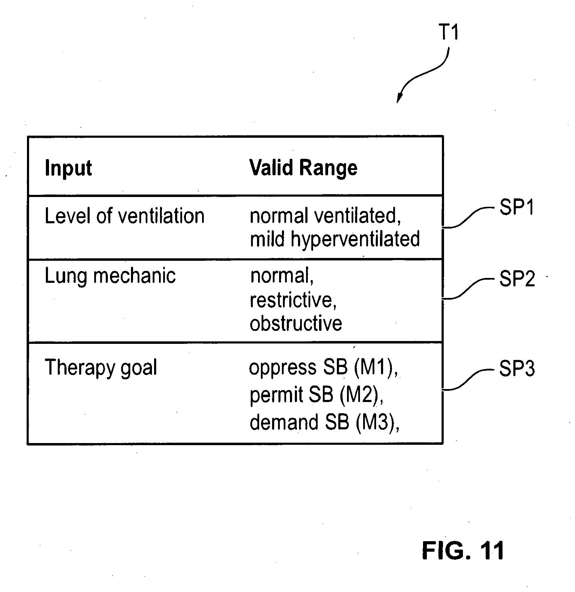

Figur 11 zeigt in der Tabelle T1 Möglichkeiten verschiedener Eingaben im Rahmen des Schrittes S1 der Figur 6. In der ersten Spalte SP1 finden sich verschiedene Einträge bzw. Vorgaben, mittels derer ein gewünschter Ventilationsgrad bzw. eine gewünschte Gasaustauschrate eines Patienten ausgewählt werden kann. Hierbei handelt es sich vorzugsweise um die Varianten einer normalen Ventilation bzw. einer milden Hyperventilation.

In Figur 11 finden sich in der zweiten Spalte SP2 ferner verschiedene Möglichkeiten von Vorgaben bezüglich einer Lungeneigenschaft ("Lung Mechanic") des Patienten. Die Vorgabe kann eine Lungeneigenschaft einer normalen, einer restriktiven oder einer obstruktiven Lunge indizieren.

Ferner zeigt Figur 11 in der dritten Spalte SP3 Möglichkeiten von Vorgaben, mittels derer eine Auswahl einer der drei Ausführungsformen des nicht erfindungsgemäßen Verfahrens vorgegeben werden kann. Eine Auswahl des Unterdrückens einer Spontanatmung "oppress spontaneous breathing (SB)" indiziert eine Auswahl der ersten Ausführungsform M1 des nicht erfindungsgemäßen Verfahrens, bei welcher im Zuge einer reinen druckgesteuerten Beatmung Spontanatemversuche des Patienten unterdrückt werden. Eine Auswahl des Erlaubens einer Spontanatmung "permit spontaneous breathing (SB)" indiziert eine Auswahl der zweiten Ausführungsform M2 des nicht erfindungsgemäßen Verfahrens, bei welcher im Zuge einer druckgesteuerten Beatmung Spontanatemversuche des Patienten erlaubt werden.

Eine Auswahl des Verlangens einer Spontanatmung "demand spontaneous breathing (SB)" indiziert eine Auswahl der dritten Ausführungsform M3 des nicht erfindungsgemäßen Verfahrens, bei welcher im Zuge einer rein druckunterstützenden Beatmung Spontanatemversuche des Patienten zwingend gefordert bzw. vorausgesetzt werden.

Anstelle der Bezeichnung "oppress spontaneous breathing (SB)" kann auch eine alternative Bezeichnung "Controlled Ventilation" gegeben sein. Anstelle der Bezeichnung "permit spontaneous breathing (SB)" kann auch eine alternative Bezeichnung "Augmented Ventilation" gegeben sein. Anstelle der Bezeichnung "demand spontaneous breathing (SB)" kann auch eine alternative Bezeichnung "Forced Spontaneous Breathing" gegeben sein.

Vorzugsweise ist die Recheneinheit dazu ausgebildet, ein Ausgabesignal AGS an eine Anzeigeeinheit AE bereitzustellen, so dass die Anzeigeeinheit AE Informationen in optischer Darstellung anzeigt, welche eine oder mehrere der zuvor genannten Vorgaben einem Betrachter zu einer Auswahl darstellen. Vorzugsweise erfolgt die Ausgabe des Ausgabesignals AGS über eine Datenschnittstelle DAS zu dem Zwecke, dass die Anzeige der Vorgaben auf einer Anzeigeeinheit erfolgt, welche nicht integraler Bestandteil der Vorrichtung BV ist.

Eine Auswahl bzw. Selektion einer oder mehrerer der zuvor genannten Vorgaben durch einen Nutzer bzw. Kliniker kann durch die in Figur 5 gezeigte Schnittstelle EE bzw. Eingabeeinheit EE der Beatmungsvorrichtung BV entgegengenommen werden. Diese Eingabeeinheit EE kann vorzugsweise eine zu der Beatmungsvorrichtung BV zugehörige oder eine mit der Beatmungsvorrichtung BV in Kommunikation stehende Eingabeschnittstelle EE sein, wobei eine Eingabeeinheit z.B eine Tastatur, ein Touch-Screen, ein Dreh-Druck-Knop und/oder eine Computermaus ist.

Zurückkommend zur Figur 6 kann nun in dem Schritt S2 eine Initialisierung von beatmungsrelevanten Parametern vorgenommen werden. Hierbei wird die Beatmungsfrequenz RR vorzugsweise auf 12 Atemzüge/min gesetzt, die möglicherweise erforderliche Druckdifferenz $\Delta P$ auf 10 mbar, der Mindestdruck PEEP auf 5 mbar, die Triggerschwelle FT auf 3 Liter/s sowie der möglicherweise erforderliche maximale Druckwert Pinsp auf 18 mbar. Es ist für einen Fachmann klar, dass die hier gezeigten Werte nur exemplarische Werte sind und bei Ausführung des nicht erfindungsgemäßen Verfahrens sowie Realisierung der erfindungsgemäßen Vorrichtung auch anders gewählt werden können.

In einem Schritt S3 kann nun abhängig von einer Eingabe bzw. einer Selektion einer Vorgabe aus Schritt S1 hinsichtlich einer ausgewählten Ausführungsform M1, M2, M3 des nicht erfindungsgemäßen Verfahrens in einen der jeweiligen Schritte S4, S5, S6 verzweigt werden.

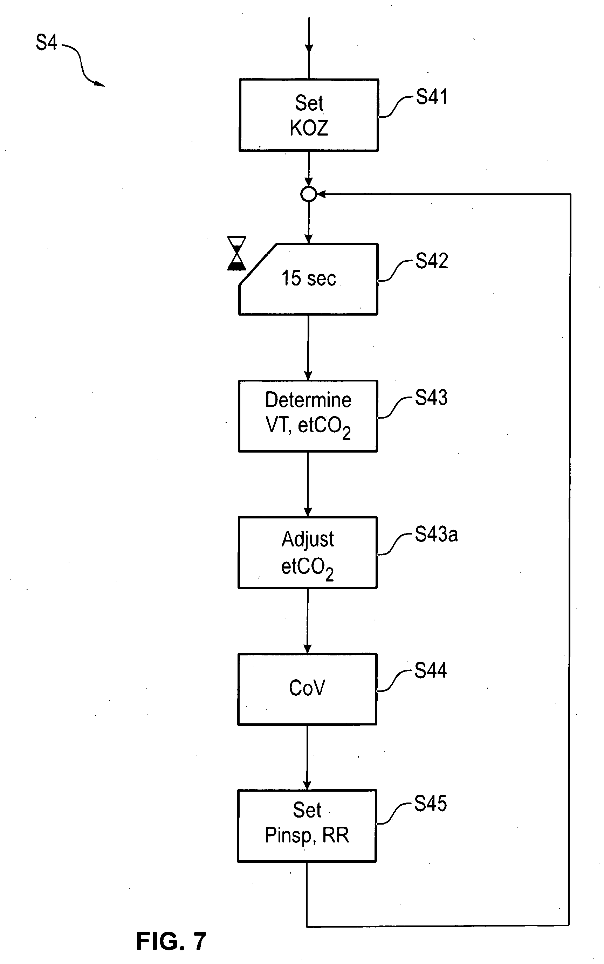

Die Figur 7 zeigt den Verfahrensschritt S4, in welchem die erste Ausführungsform M1 des nicht erfindungsgemäßen Verfahrens durchgeführt wird, wobei es sich hier um eine druckgesteuerte Beatmung ohne Zulassen von Spontanatemversuchen des Patienten handelt.

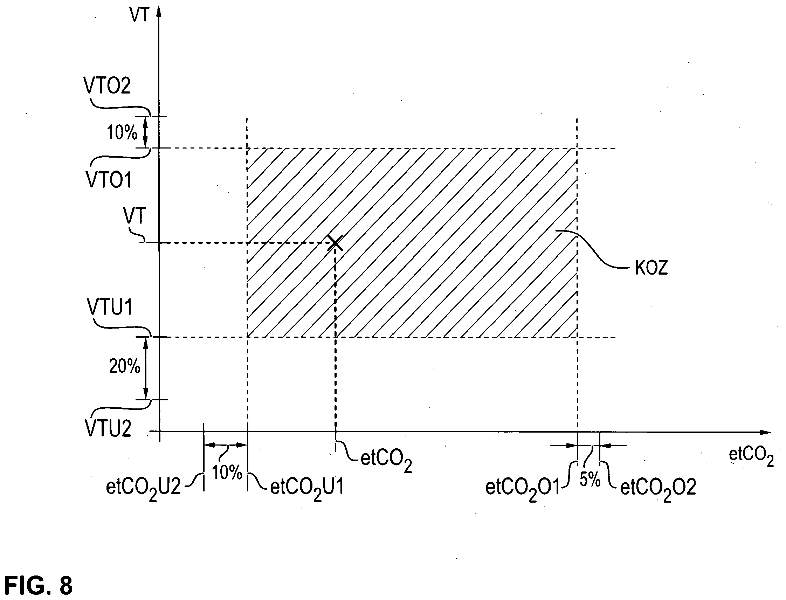

In einem ersten Teilverfahrensschritt S41 werden Grenzwerte bzw. die sogenannte Komfortzone KOZ definiert. Hierzu lässt sich in Korrespondenz zu diesem Schritt S41 die Figur 8 betrachten.

Die Figur 8 indiziert die Komfortzone KOZ, welche immer dann gegeben ist, wenn aus dem erfassten Volumenstrom durch die Recheneinheit ein durch den Patienten eingeatmetes Tidalvolumen VT ermittelt wird, welches zwischen einem oberen Volumengrenzwert VTO1 und einem unteren Volumengrenzwert VTU1 liegt. Ferner ist es zum Erreichen der Komfortzone KOZ erforderlich, dass die durch Recheneinheit R auf Basis des Kohlendioxidkonzentrationssignals KSS ermittelte endexspiratorische Kohlendioxidkonzentration etCO2 vorliegt, welche zwischen einem oberen Konzentrationsgrenzwert etCO2O1 und einem unteren Konzentrationsgrenzwert etCO2U1 liegt.

Ein möglicherweise erreichbares Ziel ist es, den Patienten derart zu beatmen, dass durch die Beatmung der Patient ein Tidalvolumen VT aufweist bzw. atmet, welches innerhalb der Volumengrenzwerte VTO1, VTU1 liegt, und dass gleichzeitig auch eine endexspiratorische Kohlendioxidkonzentration etCO2 zwischen den Konzentrationsgrenzwerten etCO2U1 und etCO2O1 liegt.

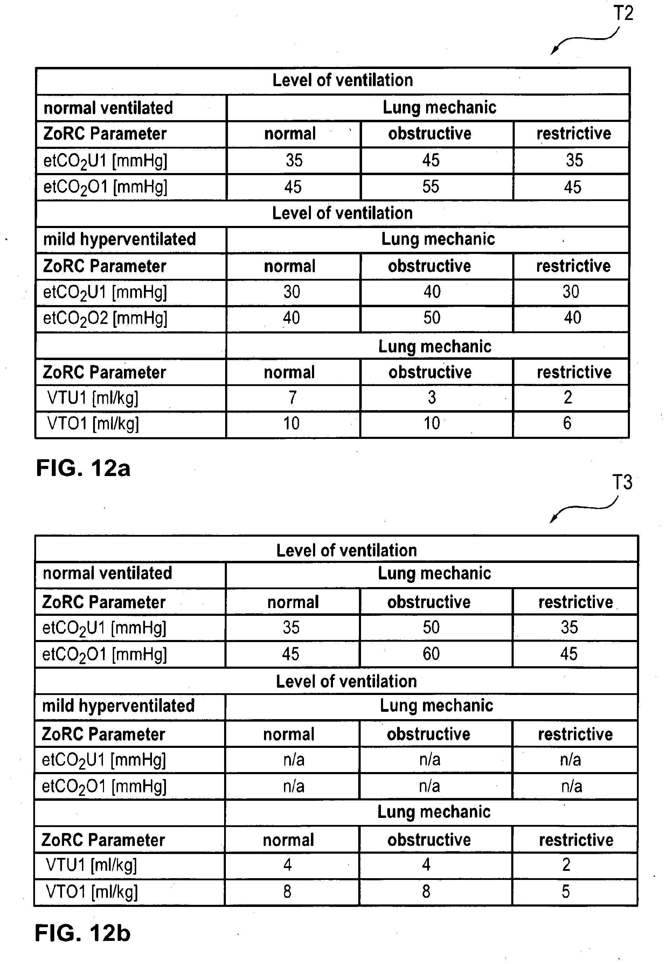

In Bezug auf den Teilschritt S41 aus Figur 7 kann unter Hinzunahme der Figur 12a und unter Betrachtung der Tabelle T2 entnommen werden, in welcher Weise durch Auswahl der Vorgaben hinsichtlich der Lungeneigenschaft des Patienten sowie der Gasaustauschrate bzw. des Ventilationsgrades die jeweiligen Volumengrenzwerte VTO1, VTU1 sowie die jeweiligen Konzentrationsgrenzwerte etCO2U1, etCO2O1 gewählt werden können.

Die Recheneinheit R der Figur 5 ermittelt auf Basis des erfassten Volumenstroms ein durch den Patienten eingeatmetes Tidalvolumen. Hierzu bestimmt die Recheneinheit vorzugsweise anhand des erfassten Volumenstroms und einer vorgegebenen Dauer Ti der inspiratorischen Phase das Tidalvolumen als Integralwert des Volumenstroms über diese Dauer Ti. Alternativ kann eine Zeitdauer einer inspiratorischen Phase derart ermittelt werden, dass bei Überschreiten eines Volumenstromschwellenwertes FT auf einen Beginn der inspiratorischen Phase geschlossen wird und bei darauffolgendem Unterschreiten des Volumenstromschwellenwertes FT auf ein Ende der inspiratorischen Phase.

Die Recheneinheit bestimmt auf Basis der erfassten Kohlendioxidkonzentration eine endexspiratorische Kohlendioxidkonzentration. Vorzugsweise wird durch Vergleich des Volumenstroms, wie in Figur 1 gezeigt, und eines unteren bzw. negativen USW Schwellenwertes dann auf ein Ende einer endexspiratorischen Phase geschlossen, wenn der erfasste Volumenstrom den unteren Schwellenwert von unten her durchschreitet.

Die durch die Recheneinheit R der Figur 5 bestimmten Werte für das Tidalvolumen sowie die endexspiratorische Kohlendioxidkonzentration werden vorzugsweise alle 4 Sekunden als Messwerte bereitgestellt. Gemäß des Teilschrittes S42 der Figur 7 wird zunächst 15 Sekunden lang gewartet und dann im Rahmen des Teilschrittes S43 das in Betracht gezogene Tidalvolumen VT mittels einer Medianfilterung jener Messwerte des Tidalvolumens ermittelt, welche in den letzten 60 Sekunden vorlagen. Ebenso wird die in Betracht gezogene endexspiratorische Kohlendioxidkonzentration etCO2 mittels einer Vorverarbeitung, vorzugsweise einer Medianfilterung, anhand jener Messwerte der endexspiratorischen Kohlendioxidkonzentration ermittelt, welche die letzten 60 Sekunden repräsentieren.

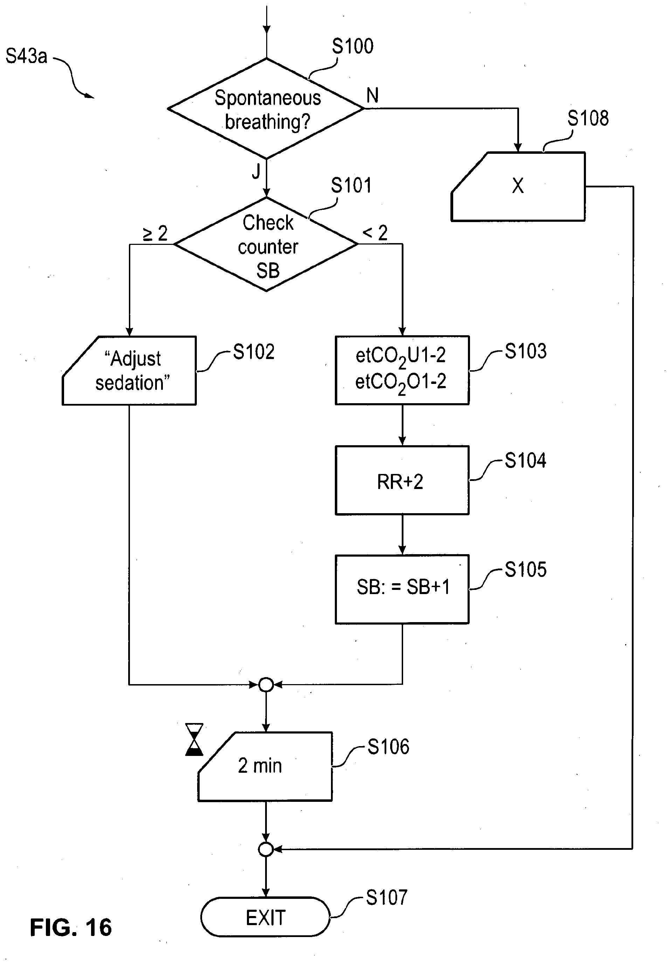

Vorzugsweise erfolgt in dem Teilschritt S43a eine Anpassung der Konzentrationsgrenzwerte. Dieses wird später in Bezug auf die Figur 16 näher erläutert.

Es wird nun zunächst erläutert, in welcher Weise eine Anpassung der Beatmungsfrequenz RR sowie eine Anpassung des Solldruckwertes Pinsp in Abhängigkeit des erfassten Volumenstromes bzw. des Tidalvolumens VT und in Abhängigkeit der erfassten Kohlendioxidkonzentration bzw. der endexspiratorischen Kohlendioxidkonzentration etCO2 durchgeführt wird. Da es sich gemäß der Ausführungsform des nicht erfindungsgemäßen Verfahrens in Bezug auf die Figur 7 um eine druckgesteuerte Beatmung handelt, wird hierbei als Solldruckwert der Druckwert Pinsp angepasst und als Beatmungsfrequenz der Wert RR.

Nach Bestimmung des Tidalvolumens VT sowie der endexspiratorischen Kohlendioxidkonzentration etCO2 im Rahmen des Teilschrittes S43 wird im Rahmen des Teilschrittes S44 ein Ventilationsgrad unter Bezug auf das Tidalvolumen bestimmt sowie ferner ein Ventilationsgrad in Bezug auf die endexspiratorische Kohlendioxidkonzentration bestimmt.

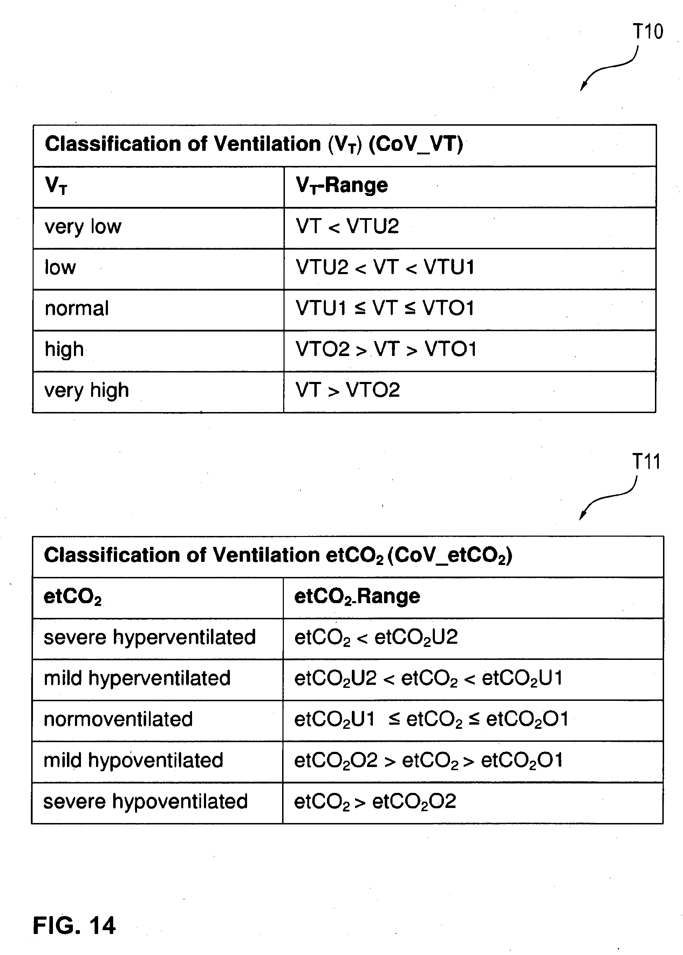

Hierzu ist die Figur 14 heranzuziehen, welche in der Tabelle T10 verschiedene Ventilationsgrade unter Vergleich des Tidalvolumens VT zu den zuvor ermittelten Grenzwerten vorgibt. Hierbei werden nicht nur der obere und der untere Grenzwert VTO1, VTU1, wie zuvor in Tabelle T2 gezeigt, herangezogen, sondern auch weitere, zweite Grenzwerte VTO2, VTU2. Diese zweiten Grenzwerte VTO2, VTU2 sind ebenso in Figur 8 eingetragen. Es werden also jeweilige zweite Grenzwerte VTO2, VTU2 verwendet, bei welchen eine Abweichung um 10% bzw. 20% von den ersten Grenzwerten VTO1, VTU1 in Betracht gezogen wird.

Entsprechendes gilt für den Grad einer Ventilation bzw. eine Gasaustauschrate in Bezug auf die endexspiratorische Kohlendioxidkonzentration in Vergleich zu den Konzentrationswerten etCO2U1, etCO2O1 sowie weiteren zweiten Konzentrationswerten etCO2U2, etCO2O2, welche um 5% bzw. 10% von den ersten Konzentrationswerten etCO2U1, etCO2O1 abweichen und ebenfalls in Figur 8 eingetragen sind.

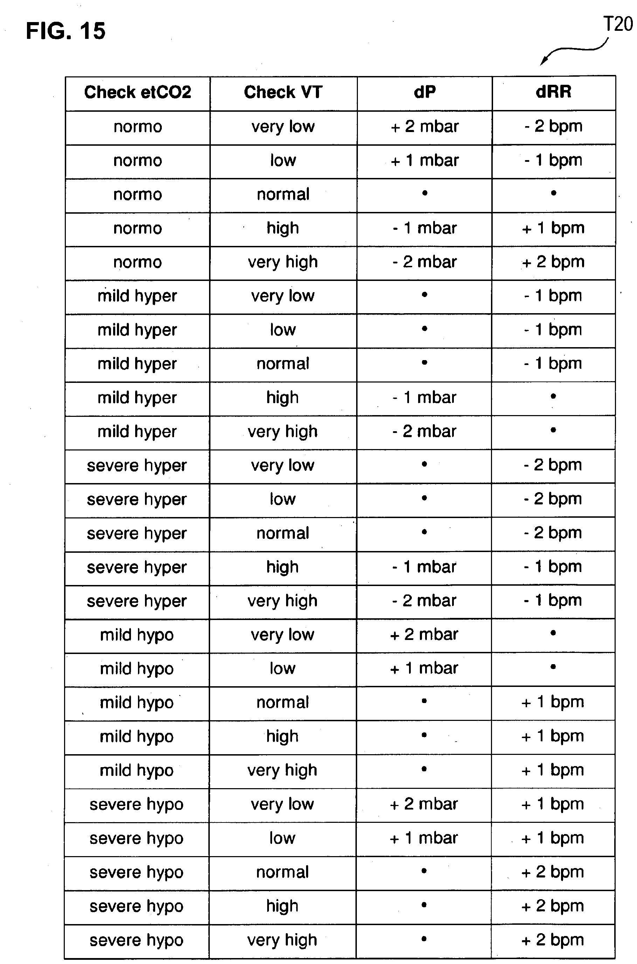

Zurückkommend zu der Figur 7 kann nun gesagt werden, dass in dem Teilschritt S45 der Solldruckwert Pinsp sowie die Beatmungsfrequenz RR angepasst werden. Dieses erfolgt unter Zuhilfenahme einer Tabelle T20 aus der Figur 15. Unter Hinzunahme der anhand von Tabelle 10 und Tabelle 11 der Figur 14 ermittelten Ventilationsgrade unter Bezug auf das Tidalvolumen VT bzw.die endexspiratorische Kohlendioxidkonzentration etCO2 kann nun anhand der Tabelle 20 der Figur 15 eine Änderung dP des Druckwertes Pinsp sowie eine Änderung dRR des Druckwertes RR ermittelt werden. Anhand der ermittelten Änderungen dP sowie dRR werden dann der Solldruckwert Pinsp sowie die Beatmungsfrequenz angepasst. Dies erfolgt gemäß

$$Pinsp := Pinsp + dP \quad ,$$

$$RR := RR + dRR$$

Das nicht erfindungsgemäße Verfahren der Figur 7 aus der Ausführungsform einer druckunterstützten Beatmung ohne Spontanatemaktivität des Patienten kehrt dann zum Teilverfahrensschritt S42 zurück.

Es wird nun erläutert, in welcher Weise in dem Teilschritt S43a vorzugsweise eine Anpassung der Konzentrationsgrenzwerte erfolgt. Hierzu zeigt die Figur 16 weitere Teilschritte des Teilschrittes S43a aus Figur 7.

Die Figur 16 zeigt Teilschritte, welche ein Teilverfahren offenbaren, bei welchem eine Spontanatemaktivität des Patienten auf Basis des erfassten Volumenstromes detektiert wird und wobei dann die Konzentrationsgrenzwerte in Abhängigkeit des Detektionsergebnisses angepasst werden. Vorzugsweise wird auch in Abhängigkeit des Detektionsergebnisses die Beatmungsfrequenz angepasst.

In dem ersten Teilschritt S100 wird überprüft, ob eine Spontanatemaktivität des Patienten vorliegt. Hierzu wird das Signal des Volumenstromsensors verwendet, wobei bei Überschreiten dieses Signals über eine Triggerschwelle, wie bereits zuvor in Bezug auf die Figur 1 erläutert, darauf geschlossen wird, dass ein Spontanatemzug bzw. eine Spontanatemaktivität des Patienten vorliegt. Da es sich in diesem Ausführungsbeispiel um einen Modus der druckgesteuerten Beatmung handelt, ist es an sich nicht vorgesehen, dass der Patient selber eine Spontanatemaktivität aufweist. Daher sollen solche Spontanatemversuche unterbunden werden und die druckgesteuerte Beatmung so durchgeführt werden, dass die Konzentrationsgrenzwerte für die endexspiratorische Kohlendioxidkonzentration in Abhängigkeit des Detektionsergebnisses aus dem Teilschritt S100 angepasst werden.

Wurde in dem Teilschritt S100 keine Spontanatemaktivität des Patienten detektiert, so wird zu einem Teilverfahrensschritt S108 verzweigt, in welchem eine möglicherweise zuvor erfolgt Ausgabe einer Warnung "Adjust Sedation", wie in Teilschritt 102 gezeigt, zurückgenommen wird. Dieses erfolgt vorzugsweise mittels eines Ausgabesignals AGS aus der Figur 5. Hierzu weist die Vorrichtung BV der Figur 5 vorzugsweise eine Anzeigeeinheit AE zur Wiedergabe des Ausgabesignals AE auf.

Es folgt dann der Teilschritt S107, welcher das Teilverfahren der Figur 16 beendet.

Wurde in dem Teilverfahrensschritt S100 eine Spontanatemaktivität des Patienten detektiert, so wird weiter verzweigt zu dem Teilverfahrensschritt S101. Hier wird ein Zähler SB überprüft, welcher vor Beginn des nicht erfindungsgemäßen Verfahrens auf den Wert 0 gesetzt wird. Ist der Zähler kleiner als ein vorgegebener Wert, beispielsweise der Wert 2, so verzweigt das Verfahren vom dem Teilverfahrensschritt S101 zu dem Teilverfahrensschritt S103. In diesem Teilverfahrensschritt S103 werden dann die Konzentrationsgrenzwerte etCO2U1 und etCO2O1 angepasst. Dieses erfolgt also abhängig davon, ob in dem Teilschritten S100 eine Spontanatemaktivität des Patienten detektiert wurde.

Vorzugsweise werden diese Konzentrationsgrenzwerte in dem Schritt 103 um einen vorgegebenen Wert verringert. Dieser Wert ist vorzugsweise der Wert 2.

Ferner erfolgt in einem darauf folgenden Teilverfahrensschritt S104 eine Anpassung der Beatmungsfrequenz RR. Vorzugsweise ist dies eine Erhöhung der Beatmungsfrequenz RR um einen vorgegebenen Wert, vorzugsweise den Wert 2.

Ferner erfolgt dann in dem Teilverfahrensschritt S105 eine Erhöhung des Zählers SB. Der Kontrollwert (2) des Zählers SB repräsentiert eine maximale Anzahl von Versuchen der Anpassung der Konzentrationsgrenzwerte etCO2U1, etCO2O2 und der Beatmungsfrequenz RR, welche angewendet werden dürfen, bevor die Warnung des Schrittes S102 ausgegeben wird. Die Warnung des Schrittes S102 indiziert das Vorliegen detektierter Spontanatemversuche des Patienten.

Es wird nach dem Teilschritt S105 weiter verfahren zu einem Teilverfahrensschritt S106, in welchem für eine vorgegeben Zeitdauer verweilt wird. Diese Zeitdauer ist vorzugsweise 2 Minuten.

Danach wird weiter verzweigt zu dem Teilverfahrensschritt S107, welcher das Teilverfahren des Schrittes S43a aus Figur 7 beendet.

Es ist also ersichtlich, dass in dem Teilverfahrensschritt S101 nur dann eine Anpassung der Konzentrationsgrenzwerte etCO2U1 und etCO2O1 in dem Teilverfahrensschritt S103 erfolgt, wenn eine maximale Anzahl von Anpassungsversuchen der Grenzwerte und der Beatmungsfrequenz (hier: 2 Versuche) noch nicht ausgeschöpft wurde. Daher wird in dem Teilverfahrensschritt S101 dann, wenn die Konzentrationsgrenzwerte und die Beatmungsfrequenz bereits mit der maximalen, vorgegebenen Anzahl an Anpassungsversuchen angepasst wurden die Warnung aus Schritt S102 ausgegeben. Es wird dann also von dem Schritt S101 hin zu dem Schritt S102 hin verzweigt, da möglicherweise die Anpassung der Konzentrationsgrenzwerte und der Beatmungsfrequenz nicht den erwünschten Erfolg erbracht haben.

In dem Teilverfahrensschritt S102 wird dann eine Ausgabe an den Kliniker vorgenommen, bei welcher der Kliniker aufgefordert wird, den Grad der Sedierung des Patienten anzupassen, um die Spontanatemaktivität des Patienten zu unterbinden.

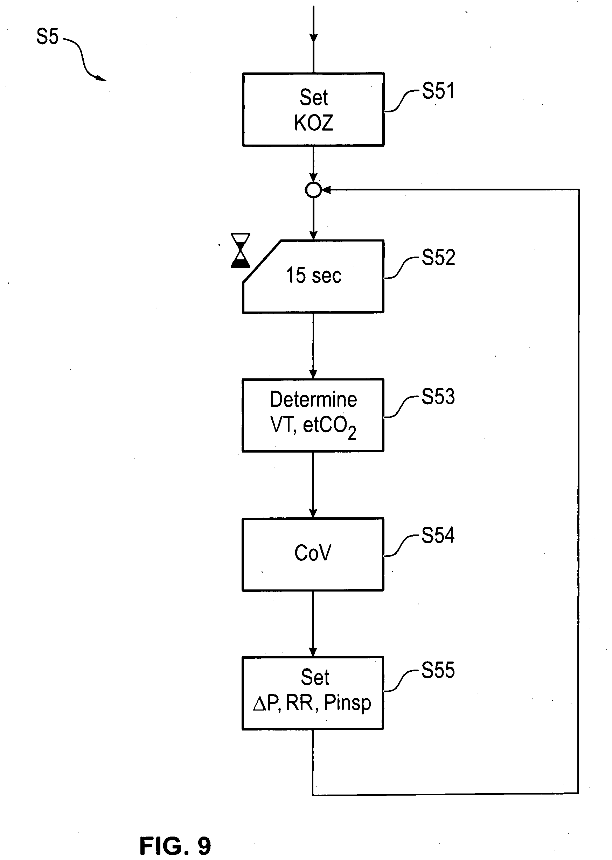

Figur 9 zeigt Teilverfahrensschritte zur Durchführung des nicht erfindungsgemäßen Verfahrens gemäß der zweiten Ausführungsform. Gemäß des ersten Teilschrittes S51 werden wiederum Grenzwerte initialisiert bzw. definiert, welche eine Komfortzone KOZ repräsentieren, wie zuvor in Figur 8 erläutert. Hierzu können aus der Tabelle T3 der Figur 12b unter Berücksichtigung der Vorgaben hinsichtlich einer Lungeneigenschaft ("Lung Mechanic") sowie einer Gasaustauschrate bzw. eines Ventilationsgrades ("Level of Ventilation") aus den Tabellen T10 und T11 der Figur 14 entsprechende erste Volumengrenzwerte VTU1, VTO2 sowie entsprechende erste Konzentrationsgrenzwerte etCO2U1, etCO2O1 ermittelt werden. Unter Verwendung der Angaben wie zuvor unter Bezug auf die Figur 8 erläutert können ferner entsprechende zweite Volumengrenzwerte VTU2, VTO2 sowie entsprechende zweite Konzentrationsgrenzwerte etCO2U2, etCO2O2 ermittelt werden.

Das nicht erfindungsgemäße Verfahren aus der Figur 9 führt eine druckgesteuerte Beatmung mit Erlauben von Spontanatemaktivität des Patienten durch, wie zuvor unter Bezug auf die Figur 3 und die Figur 1 erläutert. Eine solche druckgesteuerte Beatmung mit Erlauben einer Spontanatemaktivität verwendet somit als ersten Solldruckwert den Wert ΔP sowie vorzugsweise in Verbindung mit dem Mindestdruck PEEP, welcher während durch den Patienten getriggerter Inspirationsphasen aufgrund von Spontanatmung verwendet wird, sowie einen zweiten Solldruckwert Pinsp, welcher während Phasen der Drucksteuerung mit Triggerung aufgrund der vorgegebenen Beatmungsfrequenz RR verwendet wird. Bei der Frequenz RR handelt es sich hierbei um die Beatmungsfreuquenz welche definiert, zu welchem Zeitpunkt spätestens bzw. mit welcher Frequenz mindestens eine druckgesteuerte Beatmung des Patienten durchgeführt wird.

Nach Initialisierung der Grenzwerte in dem Teilschritt S51 wird zunächst in dem Teilschritt S52 für einen vorgegebenen Zeitraum von vorzugsweise 15 Sekunden abgewartet.

Dann erfolgt im Rahmen des Teilschrittes S53 eine Ermittlung des Tidalvolumens VT sowie der endexspiratorischen Kohlendioxidkonzentration etCO2, wie zuvor unter Bezug auf die Figur 7 und den Teilschritt S43 erläutert.

Ferner erfolgt in dem Teilschritt S54 eine Klassifikation der Ventilation bzw. Beatmung bezogen auf das Tidalvolumen VT sowie auf den endexspiratorischen Kohlendioxidkonzentrationswert etCO2, wie zuvor unter Bezug auf die Figur 7 und den Teilschritt S44 erläutert.

Im Rahmen des Teilschrittes S55 erfolgt nun eine Anpassung der beiden Solldruckwerte ∆P sowie Pinsp als auch der Beatmungsfrequenz RR. Hierzu kann unter Verwendung der Ergebnisses aus dem Teilschritt S54 bzw. den Tabellen T10 und T11 der Figur 14 anhand der Tabelle T20 der Figur 15 ein Grad der Änderung dP ermittelt werden, welcher sowohl auf den ersten Solldruckwert ∆P als auch auf den zweiten Solldruckwert Pinsp angewendet wird. Ferner kann auf Basis der Tabelle T20 der Figur 15 ein Änderungsgrad dRR für die Beatmungsfrequenz RR ermittelt werden. Dieser wird Änderungsgrad dRR wird dann auf die Beatmungsfrequenz RR angewendet.

Dann kehrt das nicht erfindungsgemäße Verfahren zurück zu dem Teilschritt S52.

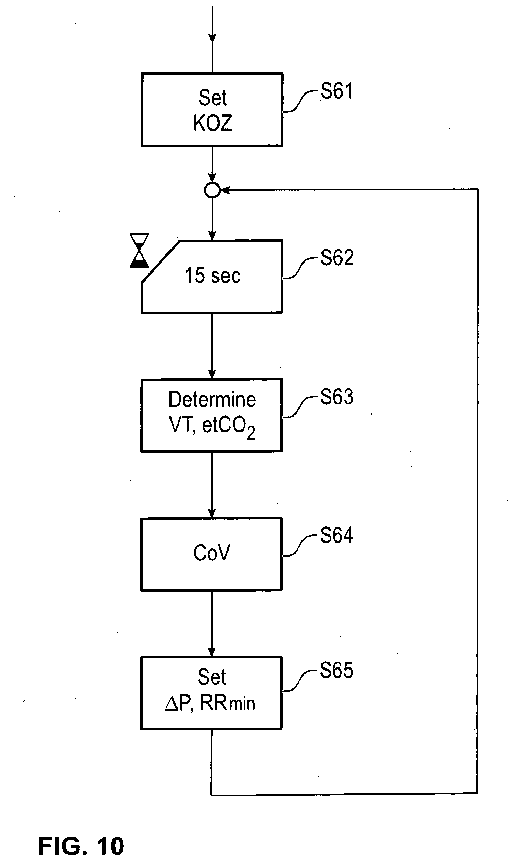

Die Figur 10 zeigt die Durchführung des nicht erfindungsgemäßen Verfahrens gemäß der dritten Ausführungsform. Hierbei handelt es sich um eine Beatmung mit Triggerung aufgrund von Spontanatemaktivität für Inspirationsphasen mit Druckunterstützung, wie zuvor unter Bezug auf die Figur 3 und die Figur 1 erläutert. Als Solldruckwert wird der Wert ∆P verwendet. Die Beatmungsfrequenz RRmin wird hierbei nicht zur Ansteuerung der Atemgasfördereinheit durch die Recheneinheit verwendet, sondern lediglich zur Bestimmung, ob die Recheneinheit ein Warnsignal WS ausgeben soll, wie zuvor beschrieben.

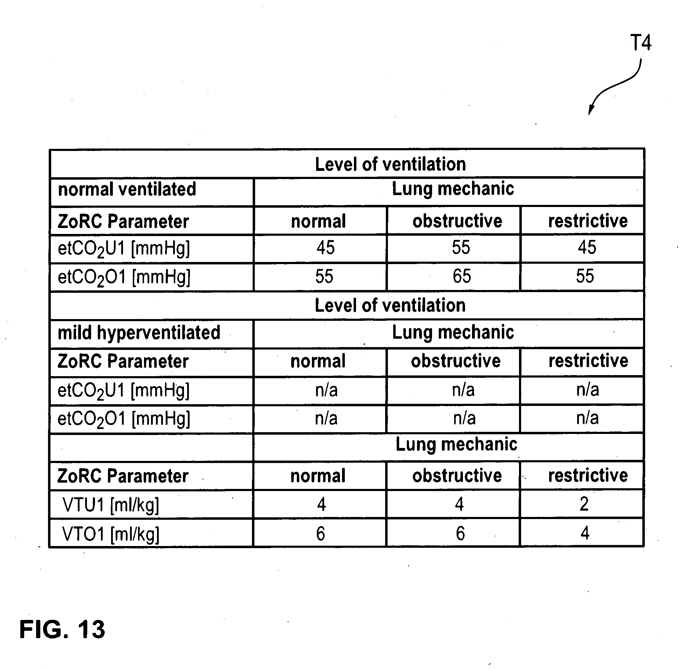

In dem Teilschritt S61 werden die zuvor beschriebenen Grenzwerte bzw. die Komfortzone unter Berücksichtigung der Vorgaben hinsichtlich der Lungeneigenschaft sowie des Ventilationsgrades bzw. der Gasaustauschrate des Patienten festgelegt, wie zuvor in Bezug auf die Schritte S41 bzw. S51 der Figuren 7 bzw. 9 erläutert. Hierzu ist die Tabelle T4 der Figur 13 heranzuziehen.

In dem Teilschritt S62 wird für einen vorgegebenen Zeitraum von vorzugsweise 15 Sekunden gewartet.

In dem Teilschritt S63 wird dann, wie zuvor unter Bezug auf die Figur 7 und den Teilschritt S43 sowie den Teilschritt S53 der Figur 9 erläutert, das Tidalvolumen VT sowie die endexspiratorischen Kohlendioxidkonzentration et $CO_2$ ermittelt.

In dem Teilschritt S64 erfolgt dann wiederum eine Klassifikation der Beatmung bzw. Ventilation in Bezug auf das Tidalvolumen VT sowie in Bezug auf die endexspiratorischen Kohlendioxidkonzentration et$CO_2$, wie zuvor unter Bezug auf die Teilverfahrensschritte S44 bzw. S54 der Figuren 7 bzw. 9 erläutert.

In Abhängigkeit der Ergebnisse aus dem Teilschritt S64 werden dann in dem Teilschritt S65 und unter Verwendung der Tabelle T20 der Figur 15 ein Druckänderungsmaß dP sowie ein Frequenzänderungsmaß dRR ermittelt. Der Solldruckwert ∆P wird dann um das Druckänderungsmaß dP geändert. Die Frequenz RRmin wird dann um das Frequenzänderungsmaß dRR geändert.

Anschließend kehrt das nicht erfindungsgemäße Verfahren zu dem Schritt S62 zurück.

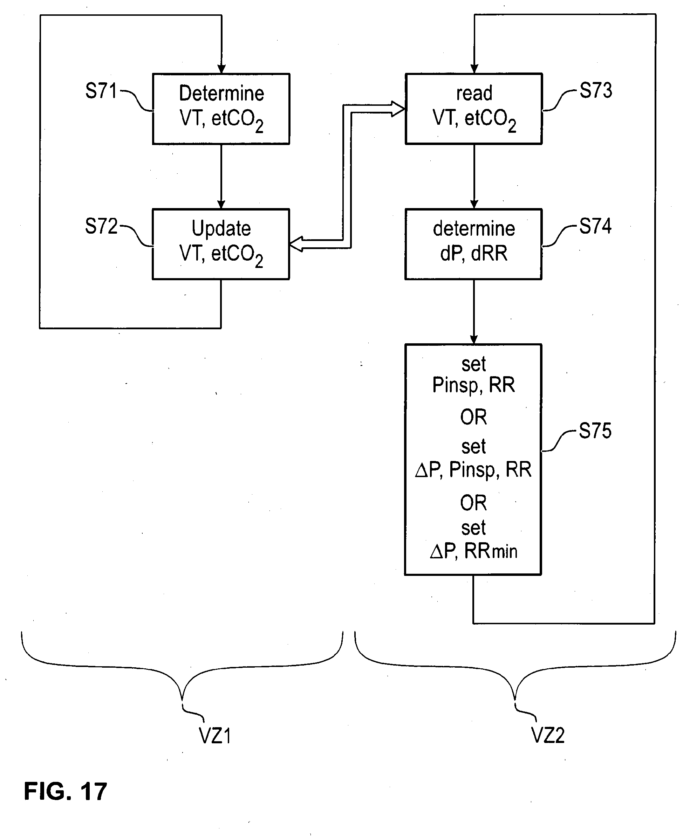

Die Figur 17 zeigt bevorzugte alternative nicht erfindungsgemäße Teilverfahrensschritte für den Fall, dass die Anpassung des Solldruckwertes, Pinsp und/oder ∆P, und die Anpassung der Beatmungsfrequenz, RR oder RRmin, in Abhängigkeit des bestimmten Tidalvolumens, eines Zieltidalvolumens, der bestimmten endexspiratorischen Kohlendioxidkonzentration und einer Ziel-Kohlendioxidkonzentration vorgenommen wird.

Die anhand der Figur 17 nun genauer erläuterte Änderung des Solldruckwertes und der Beatmungsfrequenz kann alternativ zu einer der drei Ausführungsformen M1, M2 oder M3 aus der Figur 6, welche jeweils anhand der Figuren 7, 9 bzw. erläutert wurden, durchgeführt werden, wobei in Abhängigkeit davon, ob eine rein druckgesteuerte Beatmung, eine druckgesteuerte Beatmung mit Zulassen von Spontanatemversuchen oder eine rein druckunterstützende Beatmung mit Zulassen bzw. Fordern von Spontanatemversuchen erfolgt, in dem Schritt S75 unterschiedliche Parameter angepasst werden.

Bei einer rein druckgesteuerten Beatmung werden im Schritt S75 die bekannten Parameter des Solldruckwertes Pinsp und der Beatmungsfrequenz RR in Abhängigkeit des gemessenen Tidalvolumens VT und der endexspiratorischen Kohlendioxidkonzentration et$CO_2$ angepasst. Bei einer druckgesteuerten Beatmung mit Spontanatemversuchen werden im Schritt S75 die Parameter der Solldruckwerte Pinsp und ∆P sowie die Beatmungsfrequenz RR in Abhängigkeit des gemessenen Tidalvolumens VT und der endexspiratorischen Kohlendioxidkonzentration et$CO_2$ angepasst. Bei einer rein druckunterstützenden Beatmung werden im Schritt S75 die Parameter des Solldruckwertes ∆P und der Beatmungsfrequenz RRmin in Abhängigkeit des gemessenen Tidalvolumens VT und der endexspiratorischen Kohlendioxidkonzentration et$CO_2$ angepasst.

In einem ersten Verfahrenszweig VZ1 laufen aufeinanderfolgend die Schritte S71 und S72 ab. In dem Schritt S71 werden auf Basis des Volumenstromsignals und des Kohlendioxidkonzentrationssignals ein gemessenes Tidalvolumen VT und eine endexspiratorische Kohlendioxidkonzentration et$CO_2$ bestimmt.

Die Recheneinheit R der Figur 5 ermittelt auf Basis des erfassten Volumenstroms ein durch den Patienten eingeatmetes Tidalvolumen VT, vorzugsweise in mL. Hierzu bestimmt die Recheneinheit vorzugsweise anhand des erfassten Volumenstroms und einer vorgegebenen Dauer Ti der inspiratorischen Phase das Tidalvolumen als Integralwert des Volumenstroms über diese Dauer Ti. Alternativ kann eine Zeitdauer einer inspiratorischen Phase derart ermittelt werden, dass bei Überschreiten eines Volumenstromschwellenwertes FT auf einen Beginn der inspiratorischen Phase geschlossen wird und bei darauffolgendem Unterschreiten des Volumenstromschwellenwertes FT auf ein Ende der inspiratorischen Phase.

Die Recheneinheit bestimmt ferner auf Basis der erfassten Kohlendioxidkonzentration eine endexspiratorische Kohlendioxidkonzentration etCO2, vorzugsweise in mmHg. Vorzugsweise wird durch Vergleich des Volumenstroms, wie in Figur 1 gezeigt, und eines unteren bzw. negativen USW Schwellenwertes dann auf ein Ende einer endexspiratorischen Phase geschlossen, wenn der erfasste Volumenstrom den unteren Schwellenwert von unten her durchschreitet.

In dem Verfahrensschritt S72 werden dann die ermittelten Größen VT und etCO2 aktualisiert. Dieses erfolgt vorzugsweise derart, dass die Recheneinheit R die ermittelten Größen VT und etCO2 in der Speichereinheit MEM der Figur 5 ablegt. Es kann beispielhaft angenommen werden, dass eine solche Aktualisierung der ermittelten Größen alle 1 bis 4 Sekunden erfolgt, wobei die Erfindung nicht auf diesen Wertebereich eingeschränkt ist.

In einem zu dem Verfahrenszweig VZ1 parallel ablaufenden Verfahrenszweig VZ2 werden die ermittelten Größen VT und etCO2 zunächst aus der Speichereinheit MEM der Figur 5 ausgelesen.

In einem Verfahrensschritt S74 erfolgt dann eine Bestimmung einer Druckänderung dP und einer Frequenzänderung RR. Hierbei wird ein Zieltidalvolumen VTZ von vorzugsweise VTZ=500 mL vorgegeben. Ferner wird eine Ziel-Kohlendioxidkonzentration etCO2Z von vorzugsweise etCO2Z=38 mmHg vorgegeben. Ferner wird vorzugsweise eine Compliance CL einer Lunge des Patienten von vorzugsweise CL=50 mL/mbar vorgegeben. Vorzugsweise wird ein Wirkkoeffizient DetCo2 einer Änderung der endexspiratorischen Kohlendioxidkonzentration etCO2 auf die Änderung dRR der Beatmungsfrequenz von DetCo2= - 6 mmHG / (1/min) vorgegeben.

Vorzugsweise erfolgt die Abarbeitung der Gesamtheit der drei Teilschritte S73, S74 und S75 innerhalb einer Zeit von 10 ms (millisekunden), so dass die Druckänderung dP und die Frequenzänderung alle 10 ms auf den Solldruckwert bzw. die Beatmungsfrequenz angewendet werden.

Die Druckänderung dP kann dann bestimmt werden gemäß

$$dP = \frac{VTZ - VT}{CL} \cdot \frac{10^{-3} \sec}{120 \sec}$$

Die Frequenzänderung kann dann bestimmt werden gemäß

$$dRR = \frac{etCO2Z - etCO2}{DetCO2} \cdot \frac{10^{-3} \sec}{120 \sec}$$

Der jeweils hintere Korrekturterm

$$\frac{10^{-3} \sec}{120 \sec}$$

berücksichtigt hierbei, dass in dem Zweig VZ2 die Größen dP bzw. dRR schneller geändert werden als das gemessene Tidalvolumen VT und die endexspiratorische Kohlendioxidkonzentration etCO2 in dem Zweig VZ1.

Wird eine rein druckgesteuerte Beatmung durchgeführt, so erfolgt in dem Schritt S75 die Anpassung der relevanten Parameter gemäß

$$Pinsp := Pinsp + dP \quad ,$$

$$RR := RR + dRR$$

Wird eine druckgesteuerte Beatmung mit Spontanatemversuchen durchgeführt, so erfolgt in dem Schritt S75 die Anpassung der relevanten Parameter gemäß

$$Pinsp := Pinsp + dP \quad ,$$

$$\Delta P := \Delta P + dP \quad ,$$

$$RR:=RR+dRR$$

Wird eine rein druckunterstützende Beatmung durchgeführt, so erfolgt in dem Schritt die Anpassung der relevanten Parameter gemäß

$$\Delta P:= \Delta P+dP \qquad ,$$

$$RRmin:=RRmin+dRR$$

Obwohl manche Aspekte im Zusammenhang mit einer Vorrichtung beschrieben wurden, versteht es sich, dass diese Aspekte auch eine Beschreibung des entsprechenden nicht erfindungsgemäßen Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks/Schrittes oder Details oder Merkmals einer entsprechenden Vorrichtung dar, bzw. dass die Vorrichtung oder die entsprechende Recheneinheit zur Durchführung des Verfahrensschrittes ausgebildet ist.

Die in Figur 5 gezeigte Recheneinheit R ist als wenigstens eine Recheneinheit anzusehen. Eine Umsetzung der wenigstens einen Recheneinheit R kann auch durch eine Kombination mehrerer Recheneinheiten verwirklicht werden, vorzugsweise durch Verwendung von Software in Verbindung mit Hardware. Je nach bestimmten Implementierungsanforderungen können Ausführungsbeispiele der Erfindung in Hardware und/oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardware-Komponente derart zusammenwirken können oder zusammenwirken, dass das jeweilige nicht erfindungsgemäße Verfahren durchgeführt wird.

Eine programmierbare Hardware-Komponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardware-Komponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardware-Komponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß einem Ausführungsbeispiel kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch ein Schreiben in eine oder mehrere Speicherstellen eine Aktion bewirken, veranlassen oder durchführen sowie andere Geräte, Maschinen und Komponenten ansteuern.

**Bezugszeichenliste**

| | |
|---|---|
| Anzeigeeinheit | AE |
| Anästhesiegasfortleitung | ANF |
| Atemgasfördereinheit | AGF |
| Ausgabesignal | AGS |
| Ansteuersignal | ANS |
| Atemgassensor | AS |
| Beatmungsschlauch | BS |
| Beatmungsvorrichtung | BV |
| Kohlendioxidabsorber | CA |
| Änderung des Druckwertes $\Delta P$ | dP |
| Änderungsgrad der Beatmungsfrequenz RR | dRR |
| Datenschnittstelle | DAS |
| Drucksensor | DS |
| Drucksensorsignal | DSS |
| Eingabeeinheit | EE |
| Exspiratorische Phase | EXP |
| Exspiratorischer Port | EP |
| endexspiratorischen Kohlend ioxid konzentration | etCO2 |
| oberer Konzentrationsgrenzwert | etCO2O1, etCO2O2 |
| unterer Konzentrationsgrenzwert | etCO2U1, etCO2U2 |
| Beatmungsfrequenz | F, RR |
| Mindestatemfrequenz | Fmin, RRmin |
| Triggerschwelle | FT |
| Inspiratorische Phase | INP |
| Inspiratorischer Port | IP |
| Kolbeneinheit | KE |
| Kolben | KO |
| Komfortzone | KOZ |
| Kohlendioxidkonzentrationssignal | KSS |
| Motor | M |
| Messleitung | LT |
| Messport | LTP |
| Speichereinheit | MEM |
| Modus | M1, M2, M3 |

(fortgesetzt)

| Narkosegasmischeinheit | NG |
|---|---|
| Steuersignal | NGAS |
| Druck | P |
| Patient | PT |
| Mindestdruck | PEEP |
| Maximaldruckwert | Pinsp |
| Maximaldruck | Pmax |
| Ventil | PV |
| Recheneinheit | R |
| Beatmungsfrequenz | RR |
| Beatmungsfrequenz | RRmin |
| Rückschlagventile | RV |
| Schritt | S1, S2, S3, S4, S5, S6 |
| Teilverfahrensschritt | S41, ... , S46, S51, ... , S56, S61, ... , S68 |
| Spalte | SP1, SP2, SP3 |
| Zeitlicher Abstand | T |
| Tabelle | T1, T2, T3, T4, T10, T11, T20 |
| Zeitdauer | Ti |
| Zeitfenster | Tmax |
| unterer Schwellenwert | USW |
| Volumenstrom | $\dot{V}$ |
| oberer Volumengrenzwert | VTO1, VTO2 |
| unterer Volumengrenzwert | VTU1, VTU2 |
| Ventilationsgrade | V1, ... , V5 |
| Volumenstromsensor | VS |
| Volumenstromsensorsignal | VSS |
| Tidalvolumen | VT |
| Verfahrenszweig | VZ1, VZ2 |
| Warnsignal | WS |
| Y-Stück | YS |
| Differenzdruck | $\Delta P$ |

**Patentansprüche**

1. Beatmungsvorrichtung (BV) zur automatisierten Beatmung eines Patienten (PT), aufweisend

   - einen exspiratorischen Port (EP) und einen inspiratorischen Port (IP) zum Anschluss eines dem Patienten (PT) zugewandten Beatmungsschlauches (BS) für ein Atemgas,
   - eine Atemgasfördereinheit (AGF),
   - wenigstens einen Volumenstromsensor (VS) zum Erfassen eines Volumenstroms (V) des Atemgases,

- wenigstens einen Atemgassensor (AS) zum Erfassen einer Kohlendioxidkonzentration des Atemgases,
- wenigstens einen Drucksensor (DS) zum Erfassen eines Drucks (P) des Atemgases,
- sowie wenigstens eine Recheneinheit (R),

wobei die Recheneinheit (R) dazu ausgebildet ist, die Atemgasfördereinheit (AGF) in Abhängigkeit des erfassten Drucks (P) und eines vorgegebenen Solldruckwertes (Pinsp, $\Delta$P) anzusteuern, **dadurch gekennzeichnet, dass** die Recheneinheit (R) ferner dazu ausgebildet ist,

- eine Anpassung des Solldruckwertes (Pinsp, $\Delta$P) in Abhängigkeit des erfassten Volumenstromes und in Abhängigkeit der erfassten Kohlendioxidkonzentration vorzunehmen
- und ferner eine Anpassung einer Beatmungsfrequenz (RR, RRmin) in Abhängigkeit des erfassten Volumenstromes (V) und in Abhängigkeit der erfassten Kohlendioxidkonzentration vorzunehmen.

2. Beatmungsvorrichtung (BV) nach Anspruch 1,
**dadurch gekennzeichnet, dass** die Recheneinheit (R) dazu ausgebildet ist, die Atemgasfördereinheit (AGF) in Abhängigkeit des erfassten Drucks (P), des vorgegebenen Solldruckwertes (Pinsp) und ferner der Beatmungsfrequenz (RR) anzusteuern,
und dass die Recheneinheit (R) dazu ausgebildet ist, die Atemgasfördereinheit (AGF) so anzusteuern, dass die automatisierte Beatmung als eine druckgesteuerte Beatmung durchgeführt wird.

3. Beatmungsvorrichtung (BV) nach Anspruch 1,
**dadurch gekennzeichnet, dass** die Recheneinheit (R) dazu ausgebildet ist, die Atemgasfördereinheit (AGF) so anzusteuern, dass die automatisierte Beatmung als eine druckunterstützende Beatmung durchgeführt wird,
und dass die Recheneinheit (R) dazu ausgebildet ist, in Abhängigkeit der Beatmungsfrequenz (RRmin) eine Ausgabe eines Warnsignals (WS) zu steuern.

4. Beatmungsvorrichtung (BV) nach Anspruch 1,
**dadurch gekennzeichnet, dass** die Recheneinheit (R) dazu ausgebildet ist

- auf Basis des erfassten Volumenstroms (V) ein dem Patienten (PT) zugeführtes Tidalvolumen (VT) zu bestimmen ,
- und ferner auf Basis der erfassten Kohlendioxidkonzentration eine endexspiratorische Kohlendioxidkonzentration (etCO2) zu bestimmen,

und dass die Recheneinheit (R) ferner dazu ausgebildet ist, die Anpassung des Solldruckwertes (Pinsp, $\Delta$P) und die Anpassung der Beatmungsfrequenz (RR, RRmin) in Abhängigkeit des bestimmten Tidalvolumens (VT) und der bestimmten endexspiratorischen Kohlendioxidkonzentration (etCO2) vorzunehmen.

5. Beatmungsvorrichtung (BV) nach Anspruch 4,
**dadurch gekennzeichnet, dass** Recheneinheit (R) ferner dazu ausgebildet ist, die Anpassung des Solldruckwertes (Pinsp, $\Delta$P) und die Anpassung der Beatmungsfrequenz (RR, RRmin) in Abhängigkeit des bestimmten Tidalvolumens (VT), eines Zieltidalvolumens (VTZ), der bestimmten endexspiratorischen Kohlendioxidkonzentration (etCO2) und einer Ziel-Kohlendioxidkonzentration (etCO2Z) vorzunehmen.

6. Beatmungsvorrichtung (BV) nach Anspruch 4,
**dadurch gekennzeichnet, dass** die Recheneinheit (R) dazu ausgebildet ist, die Anpassung des Solldruckwertes (Pinsp, $\Delta$P) und die Anpassung der Beatmungsfrequenz (RR, RRmin) in Abhängigkeit

- des bestimmten Tidalvolumens (VT),
- eines oberen Volumengrenzwertes (VTO1)
- und eines unteren Volumengrenzwertes (VTU1)
- der bestimmten endexspiratorischen Kohlendioxidkonzentration (etCO2),
- eines oberen Konzentrationsgrenzwertes (etCO2O1)
- und eines unteren Konzentrationsgrenzwertes (etCO2U1) vorzunehmen.

7. Beatmungsvorrichtung (BV) nach Anspruch 4,
**dadurch gekennzeichnet, dass** die Recheneinheit (R) dazu ausgebildet ist,

- die Atemgasfördereinheit (AGF) in Abhängigkeit des erfassten Drucks (P), des vorgegebenen Solldruckwertes (Pinsp, ∆P) und ferner der Beatmungsfrequenz (RR, RRmin) anzusteuern,
- ferner die Atemgasfördereinheit (AGF) so anzusteuern, dass die automatisierte Beatmung als eine druckgesteuerte Beatmung durchgeführt wird,
- ferner eine Spontanatemaktivität des Patienten (PT) auf Basis des erfassten Volumenstroms (V) zu detektieren,
- und ferner die Konzentrationsgrenzwerte (etCO2O1, etCO2U1) in Abhängigkeit des Detektionsergebnisses anzupassen.

8. Beatmungsvorrichtung (BV) nach Anspruch 4,
**dadurch gekennzeichnet, dass** die Recheneinheit (R) dazu ausgebildet ist,

- den oberen Volumengrenzwert (VTO1),
- den unteren Volumengrenzwert (VTU1),
- den oberen Konzentrationsgrenzwert (etCO2O1)
- und den unteren Konzentrationsgrenzwert (etCO2U1)

in Abhängigkeit einer Vorgabe hinsichtlich einer Lungeneigenschaft des Patienten (PT) zu wählen.

9. Beatmungsvorrichtung (BV) nach Anspruch 4,
**dadurch gekennzeichnet, dass** die Recheneinheit (R) dazu ausgebildet ist,

- den oberen Konzentrationsgrenzwert (etCO2O1)
- und den unteren Konzentrationsgrenzwert (etCO2U1) ferner in Abhängigkeit einer Vorgabe hinsichtlich einer erwünschten Gasaustauschrate des Patienten (PT) zu wählen.

10. Beatmungsvorrichtung (BV) nach Anspruch 4,
wobei der obere Volumengrenzwert ein erster oberer Volumengrenzwert (VTO1) ist,
wobei der untere Volumengrenzwert ein erster unterer Volumengrenzwert (VTU1) ist,
**dadurch gekennzeichnet, dass** die Recheneinheit (R) ferner dazu ausgebildet ist, die Anpassung des Solldruckwertes (Pinsp, ∆P) und die Anpassung der Beatmungsfrequenz (RR, RRmin) in Abhängigkeit

- eines zweiten oberen Volumengrenzwertes (VTO2)
- und eines zweiten unteren Volumengrenzwertes (VTU2)

vorzunehmen.

11. Beatmungsvorrichtung (BV) nach Anspruch 4,
wobei der obere Konzentrationsgrenzwert ein erster oberer Konzentrationsgrenzwert (etCO2O1) ist,
wobei der untere Konzentrationsgrenzwert ein erster unterer Konzentrationsgrenzwert (etCO2U1) ist,
**dadurch gekennzeichnet, dass** die Recheneinheit (R) ferner dazu ausgebildet ist, die Anpassung des Solldruckwertes (Pinsp, ∆P) und die Anpassung der Beatmungsfrequenz (RR, RRmin) in Abhängigkeit

- eines zweiten oberen Konzentrationsgrenzwertes (etCO2O2)
- und eines zweiten unteren Konzentrationsgrenzwertes (etCO2U2)

vorzunehmen.

12. Recheneinheit (R) für eine Beatmungsvorrichtung (BV) zur automatisierten Beatmung eines Patienten (PT), ausgebildet zum

- Erfassen eines Volumenstromsignals (VSS), welches einen Volumenstrom (V) eines Atemgases indiziert,
- Erfassen eines Kohlendioxidkonzentrationssignals (KSS), welches eine Kohlendioxidkonzentration des Atemgases indiziert,
- Erfassen eines Drucksignals (DSS), welches einen Druck (P) des Atemgases indiziert,
- Bereitstellen eines Ansteuersignals (ANS) für eine Atemgasfördereinheit (AGF), wobei die Recheneinheit (R) ausgebildet ist, das Ansteuersignal (ANS) in Abhängigkeit des erfassten Drucksignals (DSS) und eines vorgegebenen Solldruckwertes (Pinsp, ∆P) zu bestimmen,

**dadurch gekennzeichnet, dass** die Recheneinheit (R) ausgebildet ist,

- eine Anpassung des Solldruckwertes (Pinsp, ∆P) in Abhängigkeit des erfassten Volumenstromes und in Abhängigkeit der erfassten Kohlendioxidkonzentration vorzunehmen
- und ferner eine Anpassung einer Beatmungsfrequenz (RR, RRmin) in Abhängigkeit des erfassten Volumenstromes (V) und in Abhängigkeit der erfassten Kohlendioxidkonzentration vorzunehmen.

13. Programm mit einem Programmcode, umfassend Befehle zur Durchführung eines Verfahrens zum Betreiben einer Beatmungsvorrichtung (BV) zur automatisierten Beatmung,
das Verfahren aufweisend

- Erfassen eines Volumenstromsignals (VSS), welches einen Volumenstrom (V) eines Atemgases indiziert,
- Erfassen einer Kohlendioxidkonzentrationssignals (KSS), welches eine Kohlendioxidkonzentration des Atemgases indiziert,
- Erfassen eines Drucksignals (DSS), welches einen Druck (P) des Atemgases indiziert,
- Bereitstellen eines Ansteuersignals (ANS) für eine Atemgasfördereinheit (AGF) in Abhängigkeit des erfassten Drucksignals (DSS) und eines vorgegebenen Solldruckwertes (Pinsp, ∆P),

**gekennzeichnet durch**

- eine Anpassung des Solldruckwertes in Abhängigkeit des erfassten Volumenstromes (V) und in Abhängigkeit der erfassten Kohlendioxidkonzentration und ferner durch eine Anpassung einer Beatmungsfrequenz (RR, RRmin) in Abhängigkeit des erfassten Volumenstromes (V) und in Abhängigkeit der erfassten Kohlendioxidkonzentration,

wobei der Programmcode auf einem Computer, einem Prozessor oder einer programmierbaren Hardwarekomponente ausführbar ist.

## Claims

1. Ventilator (BV) for the automated ventilation of a patient (PT), having

- an expiratory port (EP) and an inspiratory port (IP) for the attachment of a ventilation tube (BS) directed towards the patient (PT) and supplying a breathing gas,
- a breathing gas delivery unit (AGF),
- at least one volume flow sensor (VS) for capturing a volume flow (V) of the breathing gas,
- at least one breathing gas sensor (AS) for capturing a carbon dioxide concentration of the breathing gas,
- at least one pressure sensor (DS) for capturing a pressure (P) of the breathing gas,
- and at least one computer (R),

wherein the computer (R) is configured to actuate the breathing gas delivery unit (AGF) as a function of the captured pressure (P) and of a preset desired pressure value (Pinsp, ∆P), **characterized in that** the computer (R) is moreover configured

- to effect an adaptation of the desired pressure value (Pinsp, ∆P) as a function of the captured volume flow and as a function of the captured carbon dioxide concentration
- and further to effect an adaptation of a ventilation rate (RR, RRmin) as a function of the captured volume flow (V) and as a function of the captured carbon dioxide concentration.

2. Ventilator (BV) according to Claim 1,
**characterized in that** the computer (R) is configured to actuate the breathing gas delivery unit (AGF) as a function of the captured pressure (P), the preset desired pressure value (Pinsp) and, further, the ventilation rate (RR),
and **in that** the computer (R) is configured to actuate the breathing gas delivery unit (AGF) in such a way that the automated ventilation is performed as a pressure control ventilation.

3. Ventilator (BV) according to Claim 1,
**characterized in that** the computer (R) is configured to actuate the breathing gas delivery unit (AGF) in such a way that the automated ventilation is performed as a pressure support ventilation, and **in that** the computer unit (R) is configured to control an output of a warning signal (WS) as a function of the ventilation rate (RRmin).

4. Ventilator (BV) according to Claim 1,
**characterized in that** the computer (R) is configured

- to determine, on the basis of the captured volume flow (V), a tidal volume (VT) delivered to the patient (PT),
- and further to determine, on the basis of the captured carbon dioxide concentration, an end-expiratory carbon dioxide concentration (etCO2),

and **in that** the computer (R) is further configured to effect the adaptation of the desired pressure value (Pinsp, $\Delta$P) and the adaptation of the ventilation rate (RR, RRmin) as a function of the determined tidal volume (VT) and the determined end-expiratory carbon dioxide concentration (etCO2).

5. Ventilator (BV) according to Claim 4,
**characterized in that** the computer (R) is further configured to effect the adaptation of the desired pressure value(Pinsp, $\Delta$P) and the adaptation of the ventilation rate(RR, RRmin) as a function of the determined tidal volume (VT), a target tidal volume (VTZ), the determined end-expiratory carbon dioxide concentration (etCO2) and a target carbon dioxide concentration (etCO2Z).

6. Ventilator (BV) according to Claim 4,
**characterized in that** the computer (R) is configured to effect the adaptation of the desired pressure value (Pinsp, $\Delta$P) and the adaptation of the ventilation rate (RR, RRmin) as a function of

- the determined tidal volume (VT),
- an upper volume limit value (VTO1)
- and a lower volume limit value (VTU1)
- the determined end-expiratory carbon dioxide concentration (etCO2),
- an upper concentration limit value (etCO2O1)
- and a lower concentration limit value (etCO2U1).

7. Ventilator (BV) according to Claim 4,
**characterized in that** the computer (R) is configured

- to actuate the breathing gas delivery unit (AGF) as a function of the captured pressure (P), the preset desired pressure value (Pinsp, $\Delta$P) and, further, the ventilation rate (RR, RRmin),
- further, to actuate the breathing gas delivery unit (AGF) in such a way that the automated ventilation is performed as a pressure control ventilation,
- further, to detect a spontaneous breathing activity of the patient (PT) on the basis of the captured volume flow (V),
- and, further, to adapt the concentration limit values (etCO2O1, etCO2U1) as a function of the detection result.

8. Ventilator (BV) according to Claim 4,
**characterized in that** the computer (R) is configured to select

- the upper volume limit value (VTO1),
- the lower volume limit value (VTU1),
- the upper concentration limit value (etCO2O1)
- and the lower concentration limit value (etCO2U1)

as a function of a prescription in respect of a lung property of the patient (PT).

9. Ventilator (BV) according to Claim 4,
**characterized in that** the computer (R) is configured to further select

- the upper concentration limit value (etCO2O1)
- and the lower concentration limit value (etCO2U1)

as a function of a prescription in respect of a desired gas exchange rate of the patient (PT).

10. Ventilator (BV) according to Claim 4,
wherein the upper volume limit value is a first upper volume limit value (VTO1),
wherein the lower volume limit value is a first lower volume limit value (VTU1),
**characterized in that** the computer (R) is further configured to effect the adaptation of the desired pressure value (Pinsp, ΔP) and the adaptation of the ventilation rate (RR, RRmin) as a function of

- a second upper volume limit value (VTO2)
- and a second lower volume limit value (VTU2).

11. Ventilator (BV) according to Claim 4,
wherein the upper concentration limit value is a first upper concentration limit value (etCO2O1),
wherein the lower concentration limit value is a first lower concentration limit value (etCO2U1),
**characterized in that** the computer (R) is further configured to effect the adaptation of the desired pressure value (Pinsp, ΔP) and the adaptation of the ventilation rate (RR, RRmin) as a function of

- a second upper concentration limit value (etCO2O2)
- and a second lower concentration limit value (etCO2U2).

12. Computer (R) for a ventilator (BV) for the automated ventilation of a patient (PT),
configured to

- capture a volume flow signal (VSS), which indicates a volume flow (V) of a breathing gas,
- capture a carbon dioxide concentration signal (KSS), which indicates a carbon dioxide concentration of the breathing gas,
- capture a pressure signal (DSS), which indicates a pressure (P) of the breathing gas,
- provide an actuation signal (ANS) for a breathing gas delivery unit (AGF), wherein the computer (R) is configured to determine the actuation signal (ANS) as a function of the captured pressure signal (DSS) and a preset desired pressure value (Pinsp, ΔP),

**characterized in that** the computer (R) is configured

- to effect an adaptation of the desired pressure value (Pinsp, ΔP) as a function of the captured volume flow and as a function of the captured carbon dioxide concentration
- and, further, to effect an adaptation of a ventilation rate (RR, RRmin) as a function of the captured volume flow (V) and as a function of the captured carbon dioxide concentration.

13. Program with a program code, comprising commands to carry out a method for operating a ventilator (BV) for automated ventilation, the method including

- capturing a volume flow signal (VSS), which indicates a volume flow (V) of a breathing gas,
- capturing a carbon dioxide concentration signal (KSS), which indicates a carbon dioxide concentration of the breathing gas,
- capturing a pressure signal (DSS), which indicates a pressure (P) of the breathing gas,
- providing an actuation signal (ANS) for a breathing gas delivery unit (AGF) as a function of the captured pressure signal (DSS) and a preset desired pressure value (Pinsp, ΔP),

**characterized by**

- an adaptation of the desired pressure value as a function of the captured volume flow (V) and as a function of the captured carbon dioxide concentration and, further, by an adaptation of a ventilation rate (RR, RRmin) as a function of the captured volume flow (V) and as a function of the captured carbon dioxide concentration,

wherein the program code is able to be executed on a computer, a processor or a programmable hardware component.

**Revendications**

1. Dispositif de ventilation (BV) dévolu à la ventilation automatisée d'un patient (PT), comprenant

   - un embout d'expiration (EP) et un embout d'inspiration (IP), en vue du raccordement d'un flexible de ventilation (BS) destiné à un gaz respiratoire et tourné vers le patient (PT),
   - une unité (AGF) de refoulement de gaz respiratoire,
   - au moins un capteur (VS) de débits volumiques, affecté à la détection d'un débit volumique (V) dudit gaz respiratoire,
   - au moins un capteur (AS) de gaz respiratoire, affecté à la détection d'une concentration en dioxyde de carbone dudit gaz respiratoire,
   - au moins un capteur de pression (DS), affecté à la détection d'une pression (P) dudit gaz respiratoire,
   - ainsi qu'au moins une unité de calcul (R),

   ladite unité de calcul (R) étant réalisée en vue de piloter ladite unité (AGF) de refoulement de gaz respiratoire en fonction de la pression (P) détectée, et d'une valeur de consigne préétablie (Pinsp, $\Delta$P) de la pression, **caractérisé par le fait que** l'unité de calcul (R) est réalisée, par ailleurs,

   - en vue de procéder à une adaptation de la valeur de consigne (Pinsp, $\Delta$P) de la pression, en fonction du débit volumique détecté et en fonction de la concentration en dioxyde de carbone détectée,
   - et, de surcroît, en vue de procéder à une adaptation d'une fréquence ventilatoire (RR, RRmin) en fonction dudit débit volumique (V) détecté, et en fonction de ladite concentration en dioxyde de carbone détectée.

2. Dispositif de ventilation (BV) selon la revendication 1, **caractérisé par le fait que** l'unité de calcul (R) est réalisée en vue de piloter l'unité (AGF) de refoulement de gaz respiratoire en fonction de la pression (P) détectée, de la valeur de consigne préétablie (Pinsp) de la pression et, par ailleurs, de la fréquence ventilatoire (RR) ; et **par le fait que** ladite unité de calcul (R) est réalisée en vue de piloter ladite unité (AGF) de refoulement de gaz respiratoire de façon telle que la ventilation automatisée soit exécutée en tant que ventilation à pression contrôlée.

3. Dispositif de ventilation (BV) selon la revendication 1, **caractérisé par le fait que** l'unité de calcul (R) est réalisée en vue de piloter l'unité (AGF) de refoulement de gaz respiratoire de façon telle que la ventilation automatisée soit exécutée en tant que ventilation avec aide inspiratoire ; et **par le fait que** ladite unité de calcul (R) est réalisée en vue de commander une émission d'un signal d'alerte (WS) en fonction de la fréquence ventilatoire (RRmin).

4. Dispositif de ventilation (BV) selon la revendication 1, **caractérisé par le fait que** l'unité de calcul (R) est réalisée

   - en vue de déterminer un volume courant (VT) délivré au patient (PT), sur la base du débit volumique (V) détecté
   - et, de surcroît, en vue de déterminer une concentration (etCO2) en dioxyde de carbone, en phase expiratoire finale, sur la base de la concentration en dioxyde de carbone détectée ;

   et **par le fait que** ladite unité de calcul (R) est réalisée, par ailleurs, en vue de procéder à l'adaptation de la valeur de consigne (Pinsp, $\Delta$P) de la pression, et à l'adaptation de la fréquence ventilatoire (RRmin), en fonction dudit volume courant (VT) déterminé et de ladite concentration déterminée (etCO2) en dioxyde de carbone en phase expiratoire finale.

5. Dispositif de ventilation (BV) selon la revendication 4, **caractérisé par le fait que** l'unité de calcul (R) est réalisée, par ailleurs, en vue de procéder à l'adaptation de la valeur de consigne (Pinsp, $\Delta$P) de la pression, et à l'adaptation de la fréquence ventilatoire (RR, RRmin), en fonction du volume courant (VT) déterminé, d'un volume courant (VTZ) ciblé, de la concentration déterminée (etCO2) en dioxyde de carbone en phase expiratoire finale, et d'une concentration ciblée (etCO2Z) en dioxyde de carbone.

6. Dispositif de ventilation (BV) selon la revendication 4, **caractérisé par le fait que** l'unité de calcul (R) est réalisée en vue de procéder à l'adaptation de la valeur de consigne (Pinsp, $\Delta$P) de la pression, et à l'adaptation de la fréquence ventilatoire (RR, RRmin), en fonction

- du volume courant (VT) déterminé,
- d'une valeur limite supérieure (VTO1) de volume
- et d'une valeur limite inférieure (VTU1) de volume,
- de la concentration déterminée (etCO2) en dioxyde de carbone, en phase expiratoire finale,
- d'une valeur limite supérieure (etCO2O1) de concentration
- et d'une valeur limite inférieure (etCO2U1) de concentration.

7.  Dispositif de ventilation (BV) selon la revendication 4,
**caractérisé par le fait que** l'unité de calcul (R) est réalisée en vue

- de piloter l'unité (AGF) de refoulement de gaz respiratoire en fonction de la pression (P) détectée, de la valeur de consigne préétablie (Pinsp, ΔP) de la pression et, en outre, de la fréquence ventilatoire (RR, RRmin),
- par ailleurs, de piloter ladite unité (AGF) de refoulement de gaz respiratoire de façon telle que la ventilation automatisée soit exécutée en tant que ventilation à pression contrôlée,
- de plus, de détecter une activité respiratoire spontanée du patient (PT) sur la base du débit volumique (V) détecté
- et, de surcroît, d'adapter les valeurs limites (etCO2O1, etCO2U1) de concentration en fonction du résultat de la détection.

8.  Dispositif de ventilation (BV) selon la revendication 4,
**caractérisé par le fait que** l'unité de calcul (R) est réalisée en vue de sélectionner

- la valeur limite supérieure (VTO1) de volume,
- la valeur limite inférieure (VTU1) de volume,
- la valeur limite supérieure (etCO2O1) de concentration
- et la valeur limite inférieure (etCO2U1) de concentration

en fonction d'une spécification ayant trait à une propriété pulmonaire du patient (PT).

9.  Dispositif de ventilation (BV) selon la revendication 4,
**caractérisé par le fait que** l'unité de calcul (R) est réalisée en vue de sélectionner, en outre,

- la valeur limite supérieure (etCO2O1) de concentration
- et la valeur limite inférieure (etCO2U1) de concentration

en fonction d'une spécification ayant trait à un taux d'échange gazeux souhaité du patient (PT).

10. Dispositif de ventilation (BV) selon la revendication 4,
la valeur limite supérieure de volume étant une première valeur limite supérieure (VTO1) de volume,
la valeur limite inférieure de volume étant une première valeur limite inférieure (VTU1) de volume,
**caractérisé par le fait que** l'unité de calcul (R) est réalisée, par ailleurs, en vue de procéder à l'adaptation de la valeur de consigne (Pinsp, ΔP) de la pression, et à l'adaptation de la fréquence ventilatoire (RR, RRmin), en fonction

- d'une seconde valeur limite supérieure (VTO2) de volume
- et d'une seconde valeur limite inférieure (VTU2) de volume.

11. Dispositif de ventilation (BV) selon la revendication 4,

- la valeur limite supérieure de concentration étant une première valeur limite supérieure (etCO2O1) de concentration,
- la valeur limite inférieure de concentration étant une première valeur limite inférieure (etCO2U1) de concentration,

**caractérisé par le fait que** l'unité de calcul (R) est réalisée, par ailleurs, en vue de procéder à l'adaptation de la valeur de consigne (Pinsp, ΔP) de la pression, et à l'adaptation de la fréquence ventilatoire (RR, RRmin), en fonction

- d'une seconde valeur limite supérieure (etCO2O2) de concentration
- et d'une seconde valeur limite inférieure (etCO2U2) de concentration.

**12.** Unité de calcul (R) dédiée à un dispositif de ventilation (BV) dévolu à la ventilation automatisée d'un patient (PT), réalisée en vue

- de détecter un signal (VSS) de débit volumique, indicatif d'un débit volumique (V) d'un gaz respiratoire,
- de détecter un signal (KSS) de concentration en dioxyde de carbone, indicatif d'une concentration en dioxyde de carbone dudit gaz respiratoire,
- de détecter un signal (DSS) de pression, indicatif d'une pression (P) dudit gaz respiratoire,
- de fournir un signal de pilotage (ANS) destiné à une unité (AGF) de refoulement de gaz respiratoire, ladite unité de calcul (R) étant réalisée en vue de déterminer ledit signal de pilotage (ANS) en fonction dudit signal (DSS) de pression détecté, et d'une valeur de consigne préétablie (Pinsp, ΔP) de la pression,

**caractérisée par le fait que** ladite unité de calcul (R) est réalisée en vue

- de procéder à une adaptation de la valeur de consigne (Pinsp, ΔP) de la pression en fonction du débit volumique détecté, et en fonction de la concentration en dioxyde de carbone détectée
- et, par ailleurs, en vue de procéder à une adaptation d'une fréquence ventilatoire (RR, RRmin) en fonction dudit débit volumique (V) détecté, et en fonction de ladite concentration en dioxyde de carbone détectée.

**13.** Programme comportant un code de programmation incluant des instructions en vue de la mise en œuvre d'un procédé d'actionnement d'un dispositif de ventilation (BV) dévolu à la ventilation automatisée, lequel procédé consiste à

- détecter un signal (VSS) de débit volumique, indicatif d'un débit volumique (V) d'un gaz respiratoire,
- détecter un signal (KSS) de concentration en dioxyde de carbone, indicatif d'une concentration en dioxyde de carbone dudit gaz respiratoire,
- détecter un signal (DSS) de pression, indicatif d'une pression (P) dudit gaz respiratoire,
- fournir un signal de pilotage (ANS) destiné à une unité (AGF) de refoulement de gaz respiratoire, en fonction dudit signal (DSS) de pression détecté, et d'une valeur de consigne préétablie (Pinsp, ΔP) de la pression,

**caractérisé par**

- une adaptation de la valeur de consigne de la pression, en fonction du débit volumique (V) détecté et en fonction de la concentration en dioxyde de carbone détectée et, en outre, par une adaptation d'une fréquence ventilatoire (RR, RRmin) en fonction dudit débit volumique (V) détecté, et en fonction de ladite concentration en dioxyde de carbone détectée,

sachant que le code de programmation peut être exécuté sur un ordinateur, un processeur ou un composant de matériel programmable.

**FIG. 1**

**FIG. 2**

FIG. 3

FIG. 4

P

Pinsp

ΔP

PEEP

Ti

$\frac{1}{f} \hat{=} T$

Triggerfenster zum
insp. Synchronisieren

f = RR

V̇

t

t

ohne
Spontanatmung

mit
Spontanatmung

EP 3 383 462 B1

FIG. 5

EP 3 383 462 B1

S1 ⟶ Eingaben

S2 ⟶
RR: = 12/min
ΔP: = 10 mbar
PEEP: = 5 mbar
FT: = 3 L/sec
Pinsp: = 18 mbar

S3 ⟶ Mode?

S4 ⟶ M1

S5 ⟶ M2

S6 ⟶ M3

FIG. 6

S4

```
        │
        ▼
   ┌─────────┐
   │   Set   │──── S41
   │   KOZ   │
   └─────────┘
        │
        ▼
        ○◄──────────────────────┐
        │                        │
        ▼                        │
  ▽ ┌────────┐                   │
  ▲ │ 15 sec │──── S42            │
    └────────┘                   │
        │                        │
        ▼                        │
   ┌──────────┐                  │
   │ Determine│──── S43           │
   │ VT, etCO₂│                   │
   └──────────┘                  │
        │                        │
        ▼                        │
   ┌─────────┐                   │
   │  Adjust │──── S43a           │
   │  etCO₂  │                    │
   └─────────┘                   │
        │                        │
        ▼                        │
   ┌─────────┐                   │
   │   CoV   │──── S44            │
   └─────────┘                   │
        │                        │
        ▼                        │
   ┌───────────┐                 │
   │   Set     │──── S45          │
   │ Pinsp, RR │                  │
   └───────────┘                 │
        │                        │
        └────────────────────────┘
```

**FIG. 7**

**FIG. 8**

EP 3 383 462 B1

S5

Set
KOZ — S51

15 sec — S52

Determine
VT, etCO$_2$ — S53

CoV — S54

Set
ΔP, RR, Pinsp — S55

**FIG. 9**

FIG. 10

T1

| Input | Valid Range | |
|---|---|---|
| Level of ventilation | normal ventilated, mild hyperventilated | SP1 |
| Lung mechanic | normal, restrictive, obstructive | SP2 |
| Therapy goal | oppress SB (M1), permit SB (M2), demand SB (M3), | SP3 |

FIG. 11

T2

| Level of ventilation | | | |
|---|---|---|---|
| normal ventilated | Lung mechanic | | |
| ZoRC Parameter | normal | obstructive | restrictive |
| etCO$_2$U1 [mmHg] | 35 | 45 | 35 |
| etCO$_2$O1 [mmHg] | 45 | 55 | 45 |
| Level of ventilation | | | |
| mild hyperventilated | Lung mechanic | | |
| ZoRC Parameter | normal | obstructive | restrictive |
| etCO$_2$U1 [mmHg] | 30 | 40 | 30 |
| etCO$_2$O2 [mmHg] | 40 | 50 | 40 |
| | Lung mechanic | | |
| ZoRC Parameter | normal | obstructive | restrictive |
| VTU1 [ml/kg] | 7 | 3 | 2 |
| VTO1 [ml/kg] | 10 | 10 | 6 |

## FIG. 12a

T3

| Level of ventilation | | | |
|---|---|---|---|
| normal ventilated | Lung mechanic | | |
| ZoRC Parameter | normal | obstructive | restrictive |
| etCO$_2$U1 [mmHg] | 35 | 50 | 35 |
| etCO$_2$O1 [mmHg] | 45 | 60 | 45 |
| Level of ventilation | | | |
| mild hyperventilated | Lung mechanic | | |
| ZoRC Parameter | normal | obstructive | restrictive |
| etCO$_2$U1 [mmHg] | n/a | n/a | n/a |
| etCO$_2$O1 [mmHg] | n/a | n/a | n/a |
| | Lung mechanic | | |
| ZoRC Parameter | normal | obstructive | restrictive |
| VTU1 [ml/kg] | 4 | 4 | 2 |
| VTO1 [ml/kg] | 8 | 8 | 5 |

## FIG. 12b

T4

| Level of ventilation | | | |
|---|---|---|---|
| normal ventilated | Lung mechanic | | |
| ZoRC Parameter | normal | obstructive | restrictive |
| etCO$_2$U1 [mmHg] | 45 | 55 | 45 |
| etCO$_2$O1 [mmHg] | 55 | 65 | 55 |
| Level of ventilation | | | |
| mild hyperventilated | Lung mechanic | | |
| ZoRC Parameter | normal | obstructive | restrictive |
| etCO$_2$U1 [mmHg] | n/a | n/a | n/a |
| etCO$_2$O1 [mmHg] | n/a | n/a | n/a |
| | Lung mechanic | | |
| ZoRC Parameter | normal | obstructive | restrictive |
| VTU1 [ml/kg] | 4 | 4 | 2 |
| VTO1 [ml/kg] | 6 | 6 | 4 |

# FIG. 13

T10

| Classification of Ventilation (V$_T$) (CoV_VT) | |
|---|---|
| **V$_T$** | **V$_T$-Range** |
| very low | VT < VTU2 |
| low | VTU2 < VT < VTU1 |
| normal | VTU1 ≤ VT ≤ VTO1 |
| high | VTO2 > VT > VTO1 |
| very high | VT > VTO2 |

T11

| Classification of Ventilation etCO$_2$ (CoV_etCO$_2$) | |
|---|---|
| **etCO$_2$** | **etCO$_2$.Range** |
| severe hyperventilated | etCO$_2$ < etCO$_2$U2 |
| mild hyperventilated | etCO$_2$U2 < etCO$_2$ < etCO$_2$U1 |
| normoventilated | etCO$_2$U1 ≤ etCO$_2$ ≤ etCO$_2$O1 |
| mild hypoventilated | etCO$_2$O2 > etCO$_2$ > etCO$_2$O1 |
| severe hypoventilated | etCO$_2$ > etCO$_2$O2 |

## FIG. 14

## FIG. 15

T20

| Check etCO2 | Check VT | dP | dRR |
|---|---|---|---|
| normo | very low | + 2 mbar | - 2 bpm |
| normo | low | + 1 mbar | - 1 bpm |
| normo | normal | • | • |
| normo | high | - 1 mbar | + 1 bpm |
| normo | very high | - 2 mbar | + 2 bpm |
| mild hyper | very low | • | - 1 bpm |
| mild hyper | low | • | - 1 bpm |
| mild hyper | normal | • | - 1 bpm |
| mild hyper | high | - 1 mbar | • |
| mild hyper | very high | - 2 mbar | • |
| severe hyper | very low | • | - 2 bpm |
| severe hyper | low | • | - 2 bpm |
| severe hyper | normal | • | - 2 bpm |
| severe hyper | high | - 1 mbar | - 1 bpm |
| severe hyper | very high | - 2 mbar | - 1 bpm |
| mild hypo | very low | + 2 mbar | • |
| mild hypo | low | + 1 mbar | • |
| mild hypo | normal | • | + 1 bpm |
| mild hypo | high | • | + 1 bpm |
| mild hypo | very high | • | + 1 bpm |
| severe hypo | very low | + 2 mbar | + 1 bpm |
| severe hypo | low | + 1 mbar | + 1 bpm |
| severe hypo | normal | • | + 2 bpm |
| severe hypo | high | • | + 2 bpm |
| severe hypo | very high | • | + 2 bpm |

S43a

S100

Spontaneous
breathing?

N

J

S108

X

S101

≥ 2

Check
counter
SB

< 2

S102

"Adjust
sedation"

S103

etCO$_2$U1-2
etCO$_2$O1-2

S104

RR+2

S105

SB: = SB+1

S106

2 min

S107

EXIT

**FIG. 16**

FIG. 17

**IN DER BESCHREIBUNG AUFGEFÜHRTE DOKUMENTE**

**In der Beschreibung aufgeführte Nicht-Patentliteratur**

- **KARIN DEDEN.** Beatmungsmodi in der Intensivmedizin. Dräger Medical GmbH
- Zeus Infinity Empowered. Gebrauchsanweisung. Dräger Medical AG & Co. KG, Februar 2009